# EUROPÄISCHE PATENTSCHRIFT

(11) **EP 0 942 908 B1**
(45) Veröffentlichungstag und Bekanntmachung des Hinweises auf die Patenterteilung: **13.02.2002**
(21) Anmeldenummer: 97951963.4
(22) Anmeldetag: 24.11.1997
(51) Int. Cl.: C07D 409/12, C07D 413/12, C07D 333/34, A01N 43/653

(54) **THIENYLSULFONYLAMINO(THIO)CARBONYLVERBINDUNGEN**
THIENYLSULFONYLAMINO(THIO)CARBONYL COMPOUNDS
COMPOSES THIENYLSULFONYLAMINO(THIO)CARBONYLES

(30) Priorität: 04.12.1996 DE 19650196
(43) Veröffentlichungstag der Anmeldung: 22.09.1999
(73) Patentinhaber: BAYER AG, 51368 Leverkusen (DE)
(72) Erfinder: MÜLLER, Klaus-Helmut, D-40593 Düsseldorf (DE); GESING, Ernst, Rudolf, F., D-40699 Erkrath-Hochdahl (DE); DREWES, Mark, Wilhelm, D-40764 Langenfeld (DE); JANSEN, Johannes, Rudolf, D-40789 Monheim (DE); KIRSTEN, Rolf, D-40789 Monheim (DE); KLUTH, Joachim, D-40764 Langenfeld (DE); KÖNIG, Klaus, D-51519 Odenthal (DE); PHILIPP, Ulrich, D-50674 Köln (DE); DOLLINGER, Markus, D-51381 Leverkusen (DE)
(86) Internationale Anmeldenummer: EP9706560
(87) Internationale Veröffentlichungsnummer: WO9824787

(56) Entgegenhaltungen:
- EP-A- 0 207 609
- EP-A- 0 341 489
- DE-A- 19 540 737
- US-A- 4 877 440
- US-A- 5 534 486
- US-A- 5 599 944

## Beschreibung

Die Erfindung betrifft neue Thienylsulfonylamino(thio)carbonylverbindungen, mehrere Verfahren und neue Zwischenprodukte zu ihrer Herstellung sowie ihre Verwendung als Herbizide.
Es ist bereits bekannt, daß bestimmte Sulfonylaminocarbonylverbindungen herbizide Eigenschaften aufweisen (vgl. EP 341489, EP 422469, EP 425948, EP 431291, EP 507171, EP 534266, DE 4029753, US 4 877 440, EP-A 207 609, WO 97/16 449, US 5 534 486, US 5 599 944, US 5 405 970). Die Wirkung dieser Verbindungen ist jedoch nicht in allen Belangen zufriedenstellend.
Es wurden nun die neuen Thienylsulfonylamino(thio)carbonylverbindungen der allgemeinen Formel (I), 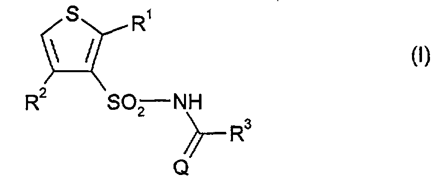 in welcher
- Q: für Sauerstoff oder Schwefel steht,
- R¹: für Cyano, Halogen oder jeweils gegebenenfalls durch Cyano, Halogen oder C₁-C₄-Alkoxy substituiertes Alkyl, Alkenyl, Alkinyl, Alkoxy, Alkenyloxy oder Alkinyloxy mit jeweils bis zu 6 Kohlenstoffatomen steht,
- R²: für Cyano, Halogen oder jeweils gegebenenfalls durch Cyano, Halogen oder C₁-C₄-Alkoxy substituiertes Alkyl, Alkenyl, Alkinyl, Alkoxy, Alkenyloxy oder Alkinyloxy mit jeweils bis zu 6 Kohlenstoffatomen steht, und
- R³: für jeweils gegebenenfalls substituiertes Heterocyclyl der nachstehenden Formeln steht 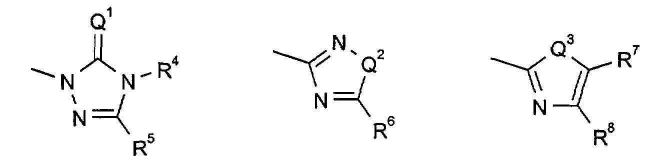
worin
- Q¹, Q² und Q³: jeweils für Sauerstoff oder Schwefel stehen sowie
- R⁴: für Wasserstoff, Hydroxy, Amino, Cyano, für C₂-C₁₀-Alkylidenamino, für gegebenenfalls durch Fluor, Chlor, Brom, Cyano, C₁-C₄-Alkoxy, C₁-C₄-Alkyl-carbonyl oder C₁-C₄-Alkoxy-carbonyl substituiertes C₁-C₆-Alkyl, für jeweils gegebenenfalls durch Fluor, Chlor und/oder Brom substituiertes C₂-C₆-Alkenyl oder C₂-C₆-Alkinyl, für jeweils gegebenenfalls durch Fluor, Chlor, Brom, Cyano, C₁-C₄-Alkoxy oder C₁-C₄-Alkoxy-carbonyl substituiertes C₁-C₆-Alkoxy, C₁-C₆-Alkylamino oder C₁-C₆-Alkyl-carbonylamino, für C₃-C₆-Alkenyloxy, für Di-(C₁-C₄-alkyl)-amino, für jeweils gegebenenfalls durch Fluor, Chlor, Brom, Cyano und/oder C₁-C₄-Alkyl substituiertes C₃-C₆-Cycloalkyl, C₃-C₆-Cycloalkylamino oder C₃-C₆-Cycloalkyl-C₁-C₄-alkyl, oder für jeweils gegebenenfalls durch Fluor, Chlor, Brom, Cyano, Nitro, C₁-C₄-Alkyl, Trifluormethyl und/oder C₁-C₄-Alkoxy substituiertes Phenyl oder Phenyl-C₁-C₄-alkyl steht,
- R⁵: für Wasserstoff, Hydroxy, Mercapto, Amino, Cyano, Fluor, Chlor, Brom, Iod, für gegebenenfalls durch Fluor, Chlor, Brom, Cyano, C₁-C₄-Alkoxy, C₁-C₄-Alkyl-carbonyl oder C₁-C₄-Alkoxy-carbonyl substituiertes C₁-C₆-Alkyl, für jeweils gegebenenfalls durch Fluor, Chlor und/oder Brom substituiertes C₂-C₆-Alkenyl oder C₂-C₆-Alkinyl, für jeweils gegebenenfalls durch Fluor, Chlor, Cyano, C₁-C₄-Alkoxy oder C₁-C₄-Alkoxy-carbonyl substituiertes C₁-C₆-Alkoxy, C₁-C₆-Alkylthio, C₁-C₆-Alkylamino oder C₁-C₆-Alkylcarbonylamino, für C₃-C₆-Alkenyloxy, C₃-C₆-Alkinyloxy, C₃-C₆-Alkenylthio, C₃-C₆-Alkinylthio, C₃-C₆-Alkenylamino oder C₃-C₆-Alkinylamino, für Di-(C₁-C₄-alkyl)-amino, für jeweils gegebenenfalls durch Methyl und/oder Ethyl substituiertes Aziridino, Pyrrolidino, Piperidino oder Morpholino, für jeweils gegebenenfalls durch Fluor, Chlor, Brom, Cyano und/oder C₁-C₄-Alkyl substituiertes C₃-C₆-Cycloalkyl, C₅-C₆-Cycloalkenyl, C₃-C₆-Cycloalkyloxy, C₃-C₆-Cycloalkylthio, C₃-C₆-Cycloalkylamino, C₃-C₆-Cycloalkyl-C₁-C₄-alkyl, C₃-C₆-Cycloalkyl-C₁-C₄-alkoxy, C₃-C₆-Cycloalkyl-C₁-C₄-alkylthio oder C₃-C₆-Cycloalkyl-C₁-C₄-alkylamino, oder für jeweils gegebenenfalls durch Fluor, Chlor, Brom, Cyano, Nitro, C₁-C₄-Alkyl, Trifluormethyl, C₁-C₄-Alkoxy und/oder C₁-C₄-Alkoxycarbonyl substituiertes Phenyl, Phenyl-C₁-C₄-alkyl, Phenoxy, Phenyl-C₁-C₄-alkoxy, Phenylthio, Phenyl-C₁-C₄-alkylthio, Phenylamino oder Phenyl-C₁-C₄-alkylamino steht, oder
- R⁴ und R⁵: zusammen für gegebenenfalls verzweigtes Alkandiyl mit 3 bis 11 Kohlenstoffatomen stehen, ferner
- R⁶, R⁷ und R⁸: gleich oder verschieden sind und für Wasserstoff, Cyano, Fluor, Chlor, Brom, oder für jeweils gegebenenfalls durch Fluor, Chlor, Brom oder C₁-C₄-Alkoxy substituiertes Alkyl, Alkenyl, Alkinyl, Alkoxy, Alkenyloxy, Alkinyloxy, Alkylthio, Alkenylthio, Alkinylthio, Alkylsulfinyl oder Alkylsulfonyl mit jeweils bis zu 6 Kohlenstoffatomen oder für gegebenenfalls durch Cyano, Fluor, Chlor, Brom oder C₁-C₄-Alkyl substituiertes Cycloalkyl mit 3 bis 6 Kohlenstoffatomen stehen,
sowie Salze von Verbindungen der Formel (I) gefunden.

Man erhält die neuen Thienylsulfonylamino(thio)carbonylverbindungen der allgemeinen Formel (I), wenn man
(a) Sulfonamide der allgemeinen Formel (II) 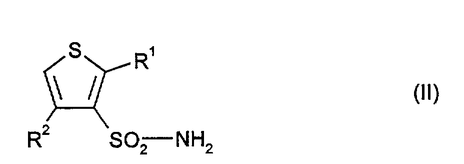 in welcher
   - R¹ and R²: die oben angegebene Bedeutung haben,
   mit (Thio)Carbonsäurederivaten der allgemeinen Formel (III) 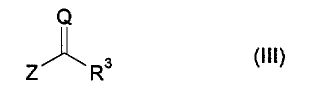 in welcher
   - Q und R³: die oben angegebene Bedeutung haben und
   - Z: für Halogen, Alkoxy, Aryloxy oder Arylalkoxy steht,
   gegebenenfalls in Gegenwart eines Säureakzeptors und gegebenenfalls in Gegenwart eines Verdünnungsmittels umsetzt,
   oder wenn man
(b) Sulfonyliso(thio)cyanate der allgemeinen Formel (IV) 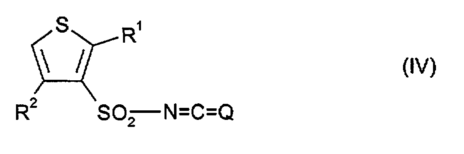 in welcher
   - Q, R¹ und R²: die oben angegebene Bedeutung haben,
   mit Heterocyclen der allgemeinen Formel (V)

   H-R³⁻¹ (V)

   in welcher
   - R³⁻¹: für gegebenenfalls substituiertes Triazolinyl der nachstehenden Formel 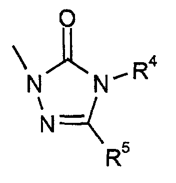 worin Q¹ für Sauerstoff oder Schwefel steht und die Reste R⁴ und R⁵ die oben angegebene Bedeutung haben,
   gegebenenfalls in Gegenwart eines Reaktionshilfsmittels und gegebenenfalls in Gegenwart eines Verdünnungsmittels umsetzt,
   oder wenn man
(c) Sulfonsäurechloride der allgemeinen Formel (VI) 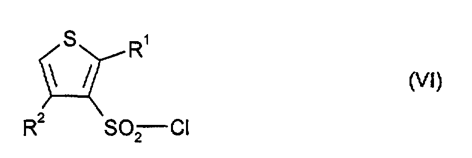 in welcher
   - R¹ und R²: die oben angegebene Bedeutung haben,
   mit Heterocyclen der allgemeinen Formel (V)

   H-R³⁻¹ (V)

   in welcher
   - R³⁻¹: die oben bei (b)angegebene Bedeutung hat,
   und Metall(thio)cyanaten der allgemeinen Formel (VII)

   MQCN (VII)

   in welcher
   - Q: die oben angegebene Bedeutung hat und
   - M: für ein Metalläquivalent steht,
   gegebenenfalls in Gegenwart eines Reaktionshilfsmittels und gegebenenfalls in Gegenwart eines Verdünnungsmittels umsetzt,
   oder wenn man
(d) Sulfonsäurechloride der allgemeinen Formel (VI) 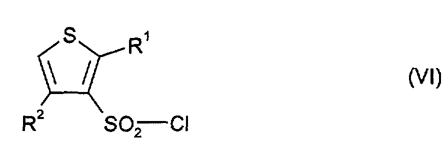 in welcher
   - R¹ und R²: die oben angegebene Bedeutung haben,
   mit (Thio)Carbonsäureamiden der allgemeinen Formel (VIII) 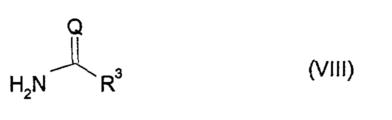 in welcher
   - Q und R³: die oben bei Formel (I) angegebene Bedeutung haben,
   gegebenenfalls in Gegenwart eines Säureakzeptors und gegebenenfalls in Gegenwart eines Verdünnungsmittels umsetzt,
   oder wenn man
(e) Sulfonylamino(thio)carbonylverbindungen der allgemeinen Formel (IX) 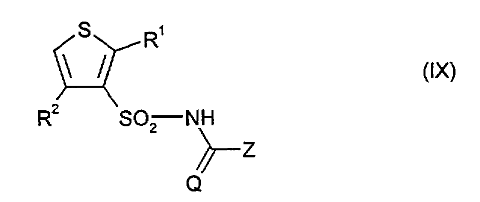 in welcher
   - Q, R¹ und R²: die oben angegebene Bedeutung haben und
   - Z: für Halogen, Alkoxy, Aryloxy oder Arylalkoxy steht,
   mit Heterocyclen der allgemeinen Formel (V)

   H-R³⁻¹ (V)

   in welcher
   - R³⁻¹: die oben bei (b) angegebene Bedeutung hat,
   gegebenenfalls in Gegenwart eines Säureakzeptors und gegebenenfalls in Gegenwart eines Verdünnungsmittels umsetzt,
und gegebenenfalls die nach den Verfahren (a), (b), (c), (d) oder (e) erhaltenen Verbindungen der Formel (I) nach üblichen Methoden in Salze überführt.

Die Verfahren (b), (c) und (e) sind naturgemäß nur zur Herstellung solcher Verbindungen der Formel (I) geeignet, bei denen R³ für R³⁻¹ steht.

Die neuen Thienylsulfonylamino(thio)carbonylverbindungen der allgemeinen Formel (I) zeichnen sich durch starke herbizide Wirksamkeit aus.

Gegenstand der Erfindung sind vorzugsweise Verbindungen der Formel (I), in welcher
- Q: für Sauerstoff oder Schwefel steht,
- R¹: für Cyano, Halogen, für gegebenenfalls durch Cyano, Halogen oder C₁-C₄-Alkoxy substituiertes C₁-C₄-Alkyl, für jeweils gegebenenfalls durch Cyano oder Halogen substituiertes C₂-C₄-Alkenyl oder C₂-C₄-Alkinyl, für gegebenenfalls durch Cyano, Halogen oder C₁-C₄-Alkoxy substituiertes C₁-C₄-Alkoxy, oder für jeweils gegebenenfalls durch Cyano oder Halogen substituiertes C₂-C₄-Alkenyloxy oder C₂-C₄-Alkinyloxy steht,
- R²: für Cyano, Halogen, für gegebenenfalls durch Cyano, Halogen oder C₁-C₄-Alkoxy substituiertes C₁-C₄-Alkyl, für jeweils gegebenenfalls durch Cyano oder Halogen substituiertes C₂-C₄-Alkenyl oder C₂-C₄-Alkinyl, für gegebenenfalls durch Cyano, Halogen oder C₁-C₄-Alkoxy substituiertes C₁-C₄-Alkoxy, oder für jeweils gegebenenfalls durch Cyano oder Halogen substituiertes C₂-C₄-Alkenyloxy oder C₂-C₄-Alkinyloxy steht, und
- R³: für jeweils gegebenenfalls substituiertes Heterocyclyl der nachstehenden Formeln steht 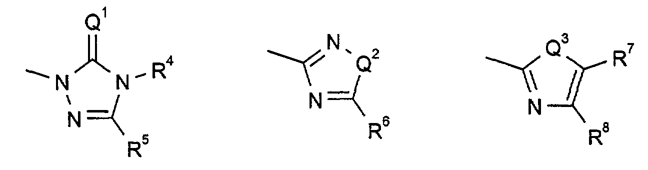
worin
- Q¹, Q² und Q³: jeweils für Sauerstoff oder Schwefel stehen sowie
- R⁴: für Wasserstoff, Hydroxy, Amino, Cyano, für C₂-C₁₀-Alkylidenamino, für gegebenenfalls durch Fluor, Chlor, Brom, Cyano, C₁-C₄-Alkoxy, C₁-C₄-Alkyl-carbonyl oder C₁-C₄-Alkoxy-carbonyl substituiertes C₁-C₆-Alkyl, für jeweils gegebenenfalls durch Fluor, Chlor und/oder Brom substituiertes C₂-C₆-Alkenyl oder C₂-C₆-Alkinyl, für jeweils gegebenenfalls durch Fluor, Chlor, Brom, Cyano, C₁-C₄-Alkoxy oder C₁-C₄-Alkoxy-carbonyl substituiertes C₁-C₆-Alkoxy, C₁-C₆-Alkylamino oder C₁-C₆-Alkyl-carbonylamino, für C₃-C₆-Alkenyloxy, für Di-(C₁-C₄-alkyl)-amino, für jeweils gegebenenfalls durch Fluor, Chlor, Brom, Cyano und/oder C₁-C₄-Alkyl substituiertes C₃-C₆-Cycloalkyl, C₃-C₆-Cycloalkylamino oder C₃-C₆-Cycloalkyl-C₁-C₄-alkyl, oder für jeweils gegebenenfalls durch Fluor, Chlor, Brom, Cyano, Nitro, C₁-C₄-Alkyl, Trifluormethyl und/oder C₁-C₄-Alkoxy substituiertes Phenyl oder Phenyl-C₁-C₄-alkyl steht,
- R⁵: für Wasserstoff, Hydroxy, Mercapto, Amino, Cyano, Fluor, Chlor, Brom, Iod, für gegebenenfalls durch Fluor, Chlor, Brom, Cyano, C₁-C₄-Alkoxy, C₁-C₄-Alkyl-carbonyl oder C₁-C₄-Alkoxy-carbonyl substituiertes C₁-C₆-Alkyl, für jeweils gegebenenfalls durch Fluor, Chlor und/oder Brom substituiertes C₂-C₆-Alkenyl oder C₂-C₆-Alkinyl, für jeweils gegebenenfalls durch Fluor, Chlor, Cyano, C₁-C₄-Alkoxy oder C₁-C₄-Alkoxy-carbonyl substituiertes C₁-C6-Alkoxy, C₁-C₆-Alkylthio, C₁-C₆-Alkylamino oder C₁-C₆-Alkylcarbonylamino, für C₃-C₆-Alkenyloxy, C₃-C₆-Alkinyloxy, C₃-C₆-Alkenylthio, C₃-C₆-Alkinylthio, C₃-C₆-Alkenylamino oder C₃-C₆-Alkinylamino, für Di-(C₁-C₄-alkyl)-amino, für jeweils gegebenenfalls durch Methyl und/oder Ethyl substituiertes Aziridino, Pyrrolidino, Piperidino oder Morpholino, für jeweils gegebenenfalls durch Fluor, Chlor, Brom, Cyano und/oder C₁-C₄-Alkyl substituiertes C₃-C₆-Cycloalkyl, C₅-C₆-Cycloalkenyl, C₃-C₆-Cycloalkyloxy, C₃-C₆-Cycloalkylthio, C₃-C₆-Cycloalkylamino, C₃-C₆-Cycloalkyl-C₁-C₄-alkyl, C₃-C₆-Cycloalkyl-C₁-C₄-alkoxy, C₃-C₆-Cycloalkyl-C₁-C₄-alkylthio oder C₃-C₆-Cycloalkyl-C₁-C₄-alkylamino, oder für jeweils gegebenenfalls durch Fluor, Chlor, Brom, Cyano, Nitro, C₁-C₄-Alkyl, Trifluormethyl, C₁-C₄-Alkoxy und/oder C₁-C₄-Alkoxycarbonyl substituiertes Phenyl, Phenyl-C₁-C₄-alkyl, Phenoxy, Phenyl-C₁-C₄-alkoxy, Phenylthio, Phenyl-C₁-C₄-alkylthio, Phenylamino oder Phenyl-C₁-C₄-alkylamino steht, oder
- R⁴ und R⁵: zusammen für gegebenenfalls verzweigtes Alkandiyl mit 3 bis 11 Kohlenstoffatomen stehen, ferner
- R⁶, R⁷ und R⁸: gleich oder verschieden sind und für Wasserstoff, Cyano, Fluor, Chlor, Brom, oder für jeweils gegebenenfalls durch Fluor, Chlor, Brom oder C₁-C₄-Alkoxy substituiertes Alkyl, Alkenyl, Alkinyl, Alkoxy, Alkenyloxy, Alkinyloxy, Alkylthio, Alkenylthio, Alkinylthio, Alkylsulfinyl oder Alkylsulfonyl mit jeweils bis zu 6 Kohlenstoffatomen oder für gegebenenfalls durch Cyano, Fluor, Chlor, Brom oder C₁-C₄-Alkyl substituiertes Cycloalkyl mit 3 bis 6 Kohlenstoffatomen stehen.

Gegenstand der Erfindung sind weiter vorzugsweise Natrium-, Kalium-, Magnesium-, Calcium-, Ammonium-, C₁-C₄-Alkyl-ammonium-, Di-(C₁-C₄-alkyl)-ammonium-, Tri-(C₁-C₄-alkyl)-ammonium-, Tetra-(C₁-C₄-alkyl)-ammonium, Tri-(C₁-C₄-alkyl)-sulfonium-, C₅- oder C₆-Cycloalkyl-ammomum- und Di-(C₁-C₂-alkyl)-benzyl-ammonium-Salze von Verbindungen der Formel (I), in welcher Q, R¹, R² und R³ die oben vorzugsweise angegebene Bedeutung haben.

Gegenstand der Erfindung sind insbesondere Verbindungen der Formel (I), in welcher
- Q: für Sauerstoff oder Schwefel steht,
- R¹: für Cyano, Fluor, Chlor, Brom, für jeweils gegebenenfalls durch Cyano, Fluor, Chlor, Methoxy oder Ethoxy substituiertes Methyl, Ethyl, n- oder i-Propyl, n-, i- oder s-Butyl, für jeweils gegebenenfalls durch Cyano, Fluor oder Chlor substituiertes Propenyl, Butenyl, Propinyl oder Butinyl, für jeweils gegebenenfalls durch Cyano, Fluor, Chlor, Methoxy oder Ethoxy substituiertes Methoxy, Ethoxy, n- oder i-Propoxy, n-, i-, s- oder t-Butoxy, oder für jeweils gegebenenfalls durch Cyano, Fluor oder Chlor substituiertes Propenyloxy, Butenyloxy, Propinyloxy oder Butinyloxy steht,
- R²: für Cyano, Fluor, Chlor, Brom, für jeweils gegebenenfalls durch Cyano, Fluor, Chlor, Methoxy oder Ethoxy substituiertes Methyl, Ethyl, n- oder i-Propyl, n-, i- oder s-Butyl, für jeweils gegebenenfalls durch Cyano, Fluor oder Chlor substituiertes Propenyl, Butenyl, Propinyl oder Butinyl, für jeweils gegebenenfalls durch Cyano, Fluor, Chlor, Methoxy oder Ethoxy substituiertes Methoxy, Ethoxy, n- oder i-Propoxy, n-, i-, s- oder t-Butoxy, oder für jeweils gegebenenfalls durch Cyano, Fluor oder Chlor substituiertes Propenyloxy, Butenyloxy, Propinyloxy oder Butinyloxy steht, und
- R³: für jeweils gegebenenfalls substituiertes Heterocyclyl der nachstehenden Formeln steht 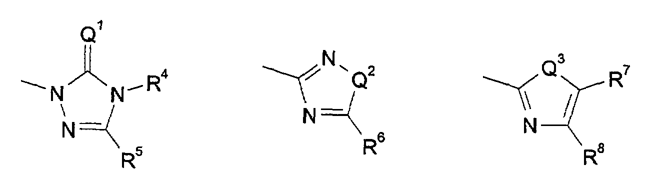
worin
- Q¹, Q² und Q³: jeweils für Sauerstoff oder Schwefel stehen sowie
- R⁴: für Wasserstoff, Hydroxy, Amino, für jeweils gegebenenfalls durch Fluor, Chlor, Cyano, Methoxy oder Ethoxy substituiertes Methyl, Ethyl, n- oder i-Propyl, n-, i-, s- oder t-Butyl, für jeweils gegebenenfalls durch Fluor, Chlor oder Brom substituiertes Propenyl, Butenyl, Propinyl oder Butinyl, für jeweils gegebenenfalls durch Fluor, Chlor, Cyano, Methoxy oder Ethoxy substituiertes Methoxy, Ethoxy, n- oder i-Propoxy, n-, i-, s- oder t-Butoxy, Methylamino, Ethylamino, n- oder i-Propylamino, n-, i-, s- oder t-Butylamino, für Propenyloxy oder Butenyloxy, für Dimethylamino oder Diethylamino, für jeweils gegebenenfalls durch Fluor, Chlor, Methyl und/oder Ethyl substituiertes Cyclopropyl, Cyclobutyl, Cyclopentyl, Cyclohexyl, Cyclopropylamino, Cyclobutylamino, Cyclopentylamino, Cyclohexylamino, Cyclopropylmethyl, Cyclobutylmethyl, Cyclopentylmethyl oder Cyclohexylmethyl, oder für jeweils gegebenenfalls durch Fluor, Chlor, Methyl, Trifluormethyl und/oder Methoxy substituiertes Phenyl oder Benzyl steht
- R⁵: für Wasserstoff, Hydroxy, Mercapto, Amino, Fluor, Chlor, Brom, für jeweils gegebenenfalls durch Fluor, Chlor, Cyano, Methoxy oder Ethoxy substituiertes Methyl, Ethyl, n- oder i-Propyl, n-, i-, s- oder t-Butyl, für jeweils gegebenenfalls durch Fluor, Chlor oder Brom substituiertes Ethenyl, Propenyl, Butenyl, Propinyl oder Butinyl, für jeweils gegebenenfalls durch Fluor, Chlor, Cyano, Methoxy oder Ethoxy substituiertes Methoxy, Ethoxy, n- oder i-Propoxy, n-, i-, s- oder t-Butoxy, Methylthio, Ethylthio, n- oder i-Propylthio, n-, i-, s- oder t-Butylthio, Methylamino, Ethylamino, n- oder i-Propylamino, n-, i-, s- oder t-Butylamino, für Propenyloxy, Butenyloxy, Propinyloxy, Butinyloxy, Propenylthio, Propadienylthio, Butenylthio, Propinylthio, Butinylthio, Propenylamino, Butenylamino, Propinylamino oder Butinylamino, für Dimethylamino, Diethylamino oder Dipropylamino, für jeweils gegebenenfalls durch Fluor, Chlor, Methyl und/oder Ethyl substituiertes Cyclopropyl, Cyclobutyl, Cyclopentyl, Cyclohexyl, Cyclopentenyl, Cyclohexenyl, Cyclopropyloxy, Cyclobutyloxy, Cyclopentyloxy, Cyclohexyloxy, Cyclopropylthio, Cyclobutylthio, Cyclopentylthio, Cyclohexylthio, Cyclopropylamino, Cyclobutylamino, Cyclopentylamino, Cyclohexylamino, Cyclopropylmethyl, Cyclobutylmethyl, Cyclopentylmethyl, Cyclohexylmethyl, Cyclopropylmethoxy, Cyclobutylmethoxy, Cyclopentylmethoxy, Cyclohexylmethoxy, Cyclopropylmethylthio, Cyclobutylmethylthio, Cyclopentylmethylthio, Cyclohexylmethylthio, Cyclopropylmethylamino, Cyclobutylmethylamino, Cyclopentylmethylamino oder Cyclohexylmethylamino, oder für jeweils gegebenenfalls durch Fluor, Chlor, Methyl, Trifluormethyl, Methoxy und/oder Methoxycarbonyl substituiertes Phenyl, Benzyl, Phenoxy, Benzyloxy, Phenylthio, Benzylthio, Phenylamino oder Benzylamino steht, oder
- R⁴ und R⁵: zusammen für gegebenenfalls verzweigtes Alkandiyl mit 3 bis 11 Kohlenstoffatomen stehen, ferner
- R⁶, R⁷ und R⁸: gleich oder verschieden sind und für Wasserstoff, Cyano, Fluor, Chlor, Brom, oder für jeweils gegebenenfalls durch Fluor, Chlor, Methoxy oder Ethoxy substituiertes Methyl, Ethyl, n- oder i-Propyl, n-, i-, s- oder t-Butyl, Propenyl, Butenyl, Propinyl, Butinyl, Methoxy, Ethoxy, n- oder i-Propoxy, n-, i-, s- oder t-Butoxy, Propenyloxy, Butenyloxy, Propinyloxy, Butinyloxy, Methylthio, Ethylthio, n- oder i-Propylthio, n-, i-, s- oder t-Butylthio, Propenylthio, Butenylthio, Propinylthio, Butinylthio, Methylsulfinyl, Ethylsulfinyl, Methylsulfonyl oder Ethylsulfonyl, oder für Cyclopropyl stehen.

Eine ganz besonders bevorzugte Gruppe erfindungsgemäßer Verbindungen sind die Verbindungen der Formel (I), in welcher
- Q: für Sauerstoff oder Schwefel steht,
- R¹: für Methyl, Ethyl, n- oder i-Propyl steht,
- R²: für Methyl, Ethyl, n- oder i-Propyl steht und
- R³: für gegebenenfalls substituiertes Triazolinyl der nachstehenden Formel steht, 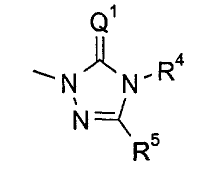
worin
- Q¹: für Sauerstoff oder Schwefel steht sowie
- R⁴: für jeweils gegebenenfalls durch Fluor, Chlor, Methoxy oder Ethoxy substituiertes Methyl, Ethyl, n- oder i-Propyl, für jeweils gegebenenfalls durch Fluor oder Chlor substituiertes Propenyl oder Propinyl, für Methoxy, Ethoxy, n- oder i-Propoxy, Methylamino, Ethylamino, n- oder i-Propylamino, für Propenyloxy, für Dimethylamino oder für Cyclopropyl steht,
- R⁵: für Chlor oder Brom, für jeweils gegebenenfalls durch Fluor, Chlor, Cyano, Methoxy oder Ethoxy substituiertes Methyl, Ethyl, n- oder i-Propyl, n-, i-, s- oder t-Butyl, für jeweils gegebenenfalls durch Fluor, Chlor oder Brom substituiertes Ethenyl, Propenyl, Butenyl, Propinyl oder Butinyl, für jeweils gegebenenfalls durch Fluor, Chlor, Cyano, Methoxy oder Ethoxy substituiertes Methoxy, Ethoxy, n- oder i-Propoxy, n-, i-, s- oder t-Butoxy, Methylthio, Ethylthio, n- oder i-Propylthio, n-, i-, s- oder t-Butylthio, Methylamino, Ethylamino, n- oder i-Propylamino, n-, i-, s- oder t-Butylamino, für Propenyloxy, Butenyloxy, Propinyloxy, Butinyloxy, Propenylthio, Propadienylthio, Butenylthio, Propinylthio, Butinylthio, Propenylamino, Butenylamino, Propinylamino oder Butinylamino, für Dimethylamino, Diethylamino oder Dipropylamino, für jeweils gegebenenfalls durch Fluor, Chlor, Methyl und/oder Ethyl substituiertes Cyclopropyl, Cyclobutyl, Cyclopentyl, Cyclohexyl, Cyclopentenyl, Cyclohexenyl, Cyclopropyloxy, Cyclobutyloxy, Cyclopentyloxy, Cyclohexyloxy, Cyclopropylthio, Cyclobutylthio, Cyclopentylthio, Cyclohexylthio, Cyclopropylamino, Cyclobutylamino, Cyclopentylamino, Cyclohexylamino, Cyclopropylmethyl, Cyclobutylmethyl, Cyclopentylmethyl, Cyclohexylmethyl, Cyclopropylmethoxy, Cyclobutylmethoxy, Cyclopentylmethoxy, Cyclohexylmethoxy, Cyclopropylmethylthio, Cyclobutylmethylthio, Cyclopentylmethylthio, Cyclohexylmethylthio, Cyclopropylmethylamino, Cyclobutylmethylamino, Cyclopentylmethylamino oder Cyclohexylmethylamino, oder für jeweils gegebenenfalls durch Fluor, Chlor, Methyl, Trifluormethyl, Methoxy und/oder Methoxycarbonyl substituiertes Phenyl, Benzyl, Phenoxy, Benzyloxy, Phenylthio, Benzylthio, Phenylamino oder Benzylamino steht, oder
- R⁴ und R⁵: zusammen für gegebenenfalls verzweigtes Alkandiyl mit 3 bis 11 Kohlenstoffatomen stehen.

Die oben aufgeführten allgemeinen oder in Vorzugsbereichen aufgeführten Restedefinitionen gelten sowohl für die Endprodukte der Formel (I) als auch entsprechend für die jeweils zur Herstellung benötigten Ausgangsstoffe bzw. Zwischenprodukte. Diese Restedefinitionen können untereinander, also auch zwischen den angegebenen bevorzugten Bereichen beliebig kombiniert werden.

Verwendet man beispielsweise 4-Brom-2-methyl-thiophen-3-sulfonamid und 5-Ethoxy-4-methyl-2-phenoxycarbonyl-2,4-dihydro-3H-1,2,4-triazol-3-thion als Ausgangsstoffe, so kann der Reaktionsablauf beim erfindungsgemäßen Verfahren (a) durch das folgende Formelschema skizziert werden:

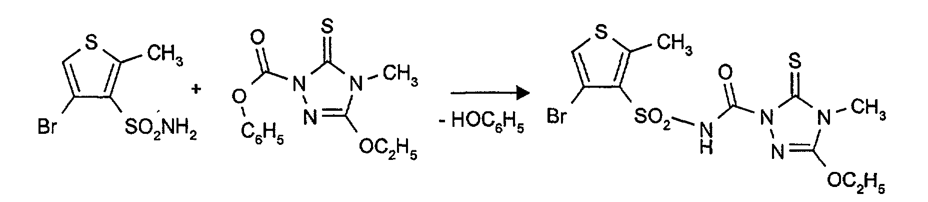

Verwendet man beispielsweise 4-Chlor-2-ethyl-3-thienylsulfonyl-isothiocyanat und 5-Ethyl-4-methoxy-2,4-dihydro-3H-1,2,4-triazol-3-on als Ausgangsstoffe, so kann der Reaktionsablauf beim erfindungsgemäßen Verfahren (b) durch das folgende Formelschema skizziert werden:

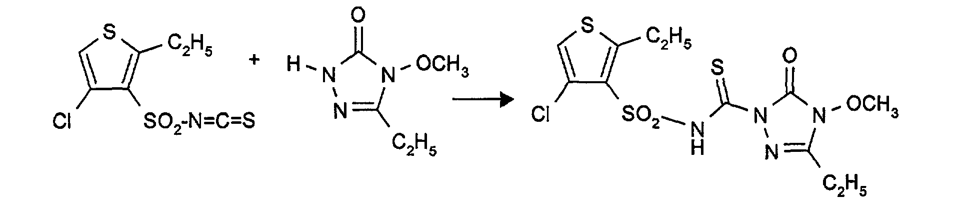

Verwendet man beispielsweise 4-Ethyl-2-methoxy-thiophen-3-sulfochlorid, 5-Ethylthio-4-methoxy-2,4-dihydro-3H-1,2,4-triazol-3-on und Kaliumcyanat als Ausgangsstoffe, so kann der Reaktionsablauf beim erfindungsgemäßen Verfahren (c) durch das folgende Formelschema skizziert werden:

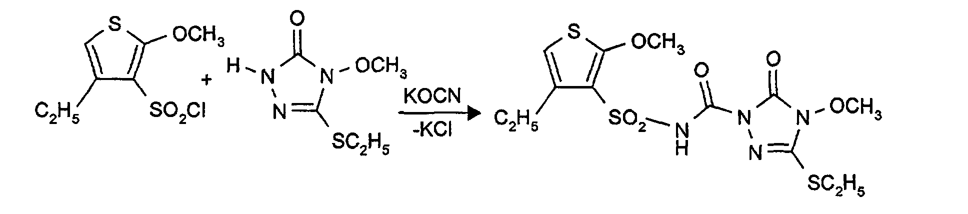

Verwendet man beispielsweise 4-Cyano-2-isopropyl-thiophen-3-sulfochlorid und 5-Methyl-1,2,4-oxadiazol-3-carboxamid als Ausgangsstoffe, so kann der Reaktionsablauf beim erfindungsgemäßen Verfahren (d) durch das folgende Formelschema skizziert werden:

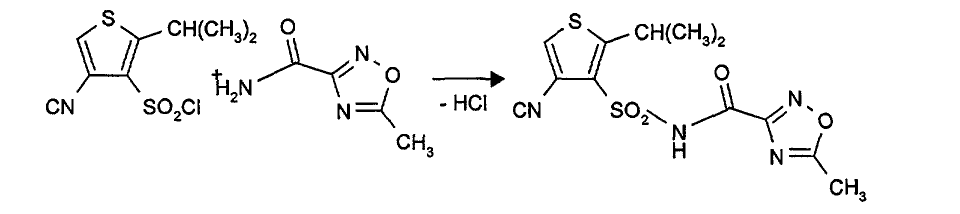

Verwendet man beispielsweise N-(4-Fluor-2-trifluormethyl-thiophen-3-yl-sulfonyl)-O-methyl-urethan und 4-Methyl-5-methylthio-2,4-dihydro-3H-1,2,4-triazol-3-on als Ausgangsstoffe, so kann der Reaktionsablauf beim erfindungsgemäßen Verfahren (e) durch das folgende Formelschema skizziert werden:

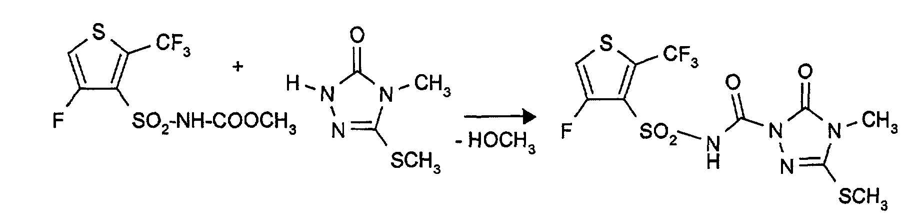

Die beim erfindungsgemäßen Verfahren (a) zur Herstellung der Verbindungen der Formel (I) als Ausgangsstoffe zu verwendenden Sulfonamide sind durch die Formel (II) allgemein definiert. In der Formel (II) haben R¹ und R² vorzugsweise bzw. insbesondere diejenige Bedeutung, die bereits oben bei der Beschreibung der erfindungsgemäßen Verbindungen der Formel (I) vorzugsweise bzw. als insbesondere bevorzugt für R¹ und R² angegeben wurde.

Die Ausgangsstoffe der Formel (II) sind noch nicht aus der Literatur bekannt; sie sind als neue Stoffe auch Gegenstand der vorliegenden Anmeldung.

Man erhält die neuen Sulfonamide der Formel (II), wenn man Sulfonsäurechloride der Formel (VI) 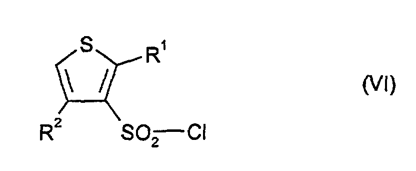 in welcher
- R¹ and R²: die oben angegebene Bedeutung haben,
mit Ammoniak, gegebenenfalls in Gegenwart eines Verdünnungsmittels, wie z.B. Wasser, bei Temperaturen zwischen 0°C und 50°C umsetzt (vgl. die Herstellungsbeispiele).

Die Sulfonsäurechloride der Formel (VI) sind ebenfalls noch nicht aus der Literatur bekannt; sie sind als neue Stoffe auch Gegenstand der vorliegenden Anmeldung.

Man erhält die neuen Sulfochloride der Formel (VI), wenn man entsprechende Aminoverbindungen der allgemeinen Formel (X) 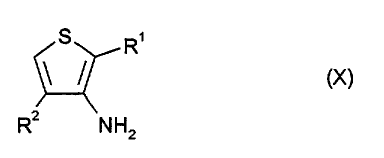 in welcher
- R¹ und R²: die oben angegebene Bedeutung haben,
mit einem Alkalimetallnitrit, wie z.B. Natriumnitrit, in Gegenwart von Salzsäure bei Temperaturen zwischen -10°C und +10°C umsetzt und die so erhaltene Diazoniumsalzlösung mit Schwefeldioxid in Gegenwart eines Verdünnungsmittels, wie z.B. Dichlormethan, 1,2-Dichlor-ethan oder Essigsäure, und in Gegenwart eines Katalysators, wie z.B. Kupfer(I)-chlorid und/oder Kupfer(II)-chlorid, bei Temperaturen zwischen -10°C und +50°C umsetzt (vgl. die Herstellungsbeispiele).

Die als Vorprodukte benötigten Aminoverbindungen der Formel (X) sind bekannt und/oder können nach an sich bekannten Verfahren hergestellt werden (vgl. DE 33 03 388).

Die beim erfindungsgemäßen Verfahren (a) zur Herstellung der Verbindungen der Formel (I) weiter als Ausgangsstoffe zu verwendenden (Thio)Carbonsäurederivate sind durch die Formel (III) allgemein definiert. In der Formel (III) haben Q und R³ vorzugsweise bzw. insbesondere diejenige Bedeutung, die bereits oben bei der Beschreibung der erfindungsgemäßen Verbindungen der Formel (I) vorzugsweise bzw. als insbesondere bevorzugt für Q und R³ angegeben wurde; Z steht vorzugsweise für Fluor, Chlor, Brom, C₁-C₄-Alkoxy, Phenoxy oder Benzyloxy, insbesondere für Chlor, Methoxy, Ethoxy oder Phenoxy.

Die Ausgangsstoffe der Formel (III) sind bekannt und/oder können nach an sich bekannten Verfahren hergestellt werden (vgl. EP 459244, EP 341489, EP 422469, EP 425948, EP 431291, EP 507171, EP 534266).

Die beim erfindungsgemäßen Verfahren (b) zur Herstellung der Verbindungen der Formel (I) als Ausgangsstoffe zu verwendenden Sulfonyliso(thio)cyanate sind durch die Formel (IV) allgemein definiert. In der Formel (IV) haben Q, R¹ und R² vorzugsweise bzw. insbesondere diejenige Bedeutung, die bereits oben bei der Beschreibung der erfindungsgemäßen Verbindungen der Formel (I) vorzugsweise bzw. als insbesondere bevorzugt für Q, R¹ und R² angegeben wurde.

Die Sulfonyliso(thio)cyanate der Formel (IV) sind noch nicht aus der Literatur bekannt; sie sind als neue Stoffe auch Gegenstand der vorliegenden Anmeldung.

Man erhält die neuen Sulfonyliso(thio)cyanate der Formel (IV), wenn man Sulfonamide der allgemeinen Formel (II) - oben - mit Phosgen bzw. Thiophosgen, gegebenenfalls in Gegenwart eines Alkylisocyanats, wie z.B. Butylisocyanat, gegebenenfalls in Gegenwart eines Reaktionshilfsmittels, wie z.B. Diazabicyclo[2.2.2]-octan, und in Gegenwart eines Verdünnungsmittels, wie z.B. Toluol, Xylol oder Chlorbenzol, bei Temperaturen zwischen 80°C und 150°C umsetzt und nach Ende der Umsetzung die flüchtigen Komponenten unter vermindertem Druck abdestilliert (vgl. die Herstellungsbeispiele).

Die bei den erfindungsgemäßen Verfahren (b), (c) und (e) als Ausgangsstoffe zu verwendenden Heterocyclen sind durch die Formel (V) allgemein definiert. In der Formel (V) steht R³⁻¹ für gegebenenfalls substituiertes Triazolinyl der nachstehenden Formel, 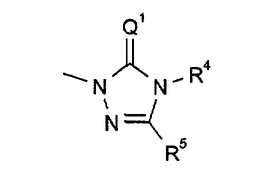 worin Q¹ für Sauerstoff oder Schwefel steht und die Reste R⁴ und R⁵ vorzugsweise bzw. insbesondere diejenigen Bedeutungen haben, die bereits oben bei der Beschreibung der erfindungsgemäßen Verbindungen der Formel (I) vorzugsweise bzw. als insbesondere bevorzugt für R⁴ bzw. R⁵ angegeben wurden.

Die Ausgangsstoffe der Formel (V) sind bekannt und/oder können nach an sich bekannten Verfahren hergestellt werden (vgl. EP 341489, EP 422469, EP 425948, EP 431291, EP 507171, EP 534266).

Die bei den erfindungsgemäßen Verfahren (c) und (d) zur Herstellung der Verbindungen der Formel (I) als Ausgangsstoffe zu verwendenden Sulfonsäurechloride sind durch die Formel (VI) allgemein definiert. In der Formel (VI) haben R¹ und R² vorzugsweise bzw. insbesondere diejenige Bedeutung, die bereits oben bei der Beschreibung der erfindungsgemäßen Verbindungen der Formel (I) vorzugsweise bzw. als insbesondere bevorzugt für R¹ und R² angegeben wurde.

Die Sulfonsäurechloride der Formel (VI) sind noch nicht aus der Literatur bekannt; sie sind als neue Stoffe auch Gegenstand der vorliegenden Anmeldung und können wie oben beschrieben hergestellt werden.

Die beim erfindungsgemäßen Verfahren (d) zur Herstellung der Verbindungen der Formel (I) als Ausgangsstoffe zu verwendenden (Thio)Carbonsäureamide sind durch die Formel (VIII) allgemein definiert. In der Formel (VIII) haben Q und R³ vorzugsweise bzw. insbesondere diejenige Bedeutung, die bereits oben bei der Beschreibung der erfindungsgemäßen Verbindungen der Formel (I) vorzugsweise bzw. als insbesondere bevorzugt für Q und R³ angegeben wurde.

Die Ausgangsstoffe der Formel (VIII) sind bekannt und/oder können nach an sich bekannten Verfahren hergestellt werden (vgl. EP 459244).

Die beim erfindungsgemäßen Verfahren (e) zur Herstellung der Verbindungen der Formel (I) als Ausgangsstoffe zu verwendenden Sulfonylamino(thio)carbonylverbindungen sind durch die Formel (IX) allgemein definiert. In der Formel (IX) haben Q, R¹ und R² vorzugsweise bzw. insbesondere diejenige Bedeutung, die bereits oben bei der Beschreibung der erfindungsgemäßen Verbindungen der Formel (I) vorzugsweise bzw. als insbesondere bevorzugt für Q, R¹ und R² angegeben wurde; Z steht vorzugsweise für Fluor, Chlor, Brom, C₁-C₄-Alkoxy, Phenoxy oder Benzyloxy, insbesondere für Chlor, Methoxy, Ethoxy oder Phenoxy.

Die Ausgangsstoffe der Formel (IX) sind bekannt und/oder können nach an sich bekannten Verfahren hergestellt werden.

Die erfindungsgemäßen Verfahren (a), (b), (c), (d) und (e) zur Herstellung der neuen Verbindungen der Formel (I) werden vorzugsweise unter Verwendung von Verdünnungsmitteln durchgeführt. Als Verdünnungsmittel kommen dabei praktisch alle inerten organischen Lösungsmittel infrage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlormethan, Chlorbenzol und o-Dichlorbenzol; Ether wie Diethyl- und Dibutylether, Glykoldimethylether und Diglykoldimethylether, Tetrahydrofuran und Dioxan; Ketone wie Aceton, Methyl-ethyl-, Methyl-isopropyl- und Methyl-isobutyl-keton; Ester wie Essigssäuremethylester und -ethylester; Nitrile wie z.B. Acetonitril und Propionitril; Amide wie z.B. Dimethylformamid, Dimethylacetamid und N-Methylpyrrolidon sowie Dimethylsulfoxid, Tetramethylensulfon und Hexamethylphosphorsäuretriamid.

Als Reaktionshilfsmittel bzw. als Säureakzeptoren können bei den erfindungsgemäßen Verfahren (a), (b), (c), (d) und (e) alle üblicherweise für derartige Umsetzungen verwendbaren Säurebindemittel eingesetzt werden. Vorzugsweise in Frage kommen Alkalimetallhydroxide wie z.B. Natrium- und Kaliumhydroxid, Erdalkalihydroxide wie z.B. Calciumhydroxid, Alkalicarbonate und -alkoholate wie Natrium- und Kaliumcarbonat, Natrium- und Kalium-tert-butylat, ferner basische Stickstoffverbindungen, wie Trimethylamin, Triethylamin, Tripropylamin, Tributylamin, Düsobutylamin, Dicyclohexylamin, Ethyldiisopropylamin, Ethyldicyclohexylamin, N,N-Dimethylbenzylamin, N,N-Dimethyl-anilin, Pyridin, 2-Methyl-, 3-Methyl-, 4-Methyl-, 2,4-Dimethyl-, 2,6-Dimethyl-, 2-Ethyl-, 4-Ethyl- und 5-Ethyl-2-methyl-pyridin, 1,5-Diazabicyclo[4,3,0]-non-5-en (DBN), 1,8-Diazabicyclo-[5,4,0]-undec-7-en (DBU) und 1,4-Diazabicyclo-[2,2,2]-octan (DABCO).

Die Reaktionstemperaturen können bei den erfindungsgemäßen Verfahren (a), (b), (c), (d) und (e) in einem größeren Bereich variiert werden. Im allgemeinen arbeitet man bei Temperaturen zwischen -20°C und +150°C, vorzugsweise bei Temperaturen zwischen 0°C und +100°C.

Die erfindungsgemäßen Verfahren (a), (b), (c), (d) und (e) werden im allgemeinen unter Normaldruck durchgeführt. Es ist jedoch auch möglich, unter erhöhtem oder vermindertem Druck zu arbeiten.

Zur Durchführung der erfindungsgemäßen Verfahren (a), (b), (c), (d) und (e) werden die jeweils benötigten Ausgangsstoffe im allgemeinen in angenähert äquimolaren Mengen eingesetzt. Es ist jedoch auch möglich, eine der jeweils eingesetzten Komponenten in einem größeren Überschuß zu verwenden. Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt bei den erfindungsgemäßen Verfahren (a), (b), (c), (d) und (e) jeweils nach üblichen Methoden (vgl. die Herstellungsbeispiele).

Aus den erfindungsgemäßen Verbindungen der allgemeinen Formel (I) können gegebenenfalls Salze hergestellt werden. Man erhält solche Salze in einfacher Weise nach üblichen Salzbildungsmethoden, beispielsweise durch Lösen oder Dispergieren einer Verbindung der Formel (I) in einem geeigneten Lösungsmittel, wie z.B. Methylenchlorid, Aceton, tert-Butyl-methylether oder Toluol, und Zugabe einer geeigneten Base. Die Salze können dann - gegebenenfalls nach längerem Rühren - durch Einengen oder Absaugen isoliert werden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:
Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.
Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.
Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.
Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Verbindungen der Formel (I) eignen sich insbesondere zur selektiven Bekämpfung von monokotylen und dikotylen Unkräutern in monokotylen Kulturen sowohl im Vorauflauf- als auch im Nachauflauf-Verfahren.

Die Wirkstoffe können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide infrage, beispielsweise

Acetochlor, Acifluorfen(-sodium), Aclonifen, Alachlor, Alloxydim(-sodium), Ametryne, Amidochlor, Amidosulfuron, Asulam, Atrazine, Azimsulfuron, Benazolin, Benfuresate, Bensulfuron(-methyl), Bentazon, Benzofenap, Benzoylprop(-ethyl), Bialaphos, Bifenox, Bromobutide, Bromofenoxim, Bromoxynil, Butachlor, Butylate, Cafenstrole, Carbetamide, Chlomethoxyfen, Chloramben, Chloridazon, Chlorimuron(-ethyl), Chlornitrofen, Chlorsulfuron, Chlortoluron, Cinmethylin, Cinosulfuron, Clethodim, Clodinafop(-propargyl), Clomazone, Clopyralid, Clopyrasulfuron, Cloransulam(-methyl), Cumyluron, Cyanazine, Cycloate, Cyclosulfamuron, Cycloxydim, Cyhalofop(-butyl), 2,4-D, 2,4-DB, 2,4-DP, Desmedipham, Diallate, Dicamba, Diclofop(-methyl), Difenzoquat, Diflufenican, Dimefuron, Dimepiperate, Dimethachlor, Dimethametryn, Dimethenamid, Dinitramine, Diphenamid, Diquat, Dithiopyr, Diuron, Dymron, EPTC, Esprocarb, Ethalfluralin, Ethametsulfüron(-methyl), Ethofumesate, Ethoxyfen, Etobenzanid, Fenoxaprop(-ethyl), Flamprop(-isopropyl), Flamprop(-isopropyl-L), Flamprop(-methyl), Flazasulfuron, Fluazifop(-butyl), Flumetsulam, Flumiclorac(-pentyl), Flumioxazin, Flumipropyn, Fluometuron, Fluorochloridone, Fluoroglycofen(-ethyl), Flupoxam, Flupropacil, Flurenol, Fluridone, Fluroxypyr, Flurprimidol, Flurtamone, Fomesafen, Glufosinate(-ammonium), Glyphosate(-isopropylammonium), Halosafen, Haloxyfop(-ethoxyethyl), Hexazinone, Imazamethabenz(-methyl), Imazamethapyr, Imazamox, Imazapyr, Imazaquin, Imazethapyr, Imazosulfuron, Ioxynil, Isopropalin, Isoproturon, Isoxaben, Isoxaflutole, Isoxapyrifop, Lactofen, Lenacil, Linuron, MCPA, MCPP, Mefenacet, Metamitron, Metazachlor, Methabenzthiazuron, Metobenzuron, Metobromuron, Metolachlor, Metosulam, Metoxuron, Metribuzin, Metsulfuron(-methyl), Molinate, Monolinuron, Naproanilide, Napropamide, Neburon, Nicosulfuron, Norflurazon Orbencarb, Oryzalin, Oxadiazon, Oxyfluorfen, Paraquat, Pendimethalin, Phenmedipham, Piperophos, Pretilachlor, Primisulfuron(-methyl), Prometryn, Propachlor, Propanil, Propaquizafop, Propyzamide, Prosulfocarb, Prosulfuron, Pyrazolate, Pyrazosulfuron(-ethyl), Pyrazoxyfen, Pyributicarb, Pyridate, Pyrithiobac(-sodium), Quinchlorac, Quinmerac, Quizalofop(-ethyl), Quizalofop(-p-tefuryl), Rimsulfuron, Sethoxydim, Simazine, Simetryn, Sulcotrione, Sulfentrazone, Sulfometuron(-methyl), Sulfosate, Tebutam, Tebuthiuron, Terbuthylazine, Terbutryn, Thenylchlor, Thiafluamide, Thiazopyr, Thidiazimin, Thifensulfuron(-methyl), Thiobencarb, Tiocarbazil, Tralkoxydim, Triallate, Triasulfuron, Tribenuron(-methyl), Triclopyr, Tridiphane, Trifluralin und Triflusulfuron.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstruktur-verbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 1 g und 10 kg Wirkstoff pro Hektar Bodenfläche, vorzugsweise zwischen 5 g und 5 kg pro ha.

Die Herstellung und die Verwendung der erfindungsgemäßen Wirkstoffe geht aus den nachfolgenden Beispielen hervor.

### Herstellungsbeispiele:

### Beispiel 1

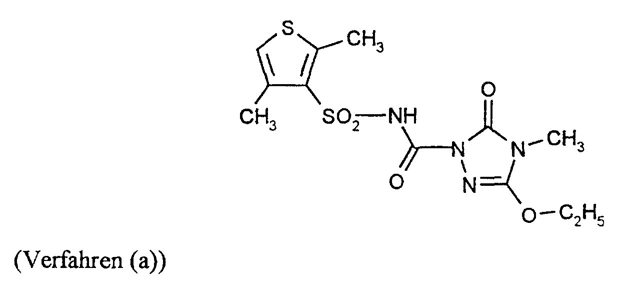

Zu einer Lösung von 1,6 g (6,4 mMol) 5-Ethoxy-4-methyl-2-phenoxycarbonyl-2,4-di-hydro-3H-1,2,4-triazol-3-on in 30 ml Acetonitril gibt man nacheinander 1,3 g (6,8 mMol) 2,4-Dimethyl-thiophen-3-sulfonamid und 1,1 g (7 mMol) 1,8-Diazabicyclo-[5,4,0]-undec-7-en (DBU). Die Reaktionsmischung wird ca. 15 Stunden bei Raumtemperatur (ca. 20°C) gerührt und anschließend im Wasserstrahlvakuum eingeengt. Der Rückstand wird dann in Methylenchlorid aufgenommen, mit 1N-Salzsäure und dann mit Wasser gewaschen. Die organische Phase wird mit Magnesiumsulfat getrocknet und filtriert. Das Filtrat wird im Wasserstrahlvakuum eingeengt und der Rückstand aus Isopropanol umkristallisiert.

Man erhält 1,1 g (50% der Theorie) 5-Ethoxy-4-methyl-2-(2,4-dimethyl-thien-3-yl-sulfonylaminocarbonyl)-2,4-dihydro-3H-1,2,4-triazol-3-on vom Schmelzpunkt 158°C.

### Beispiel 2

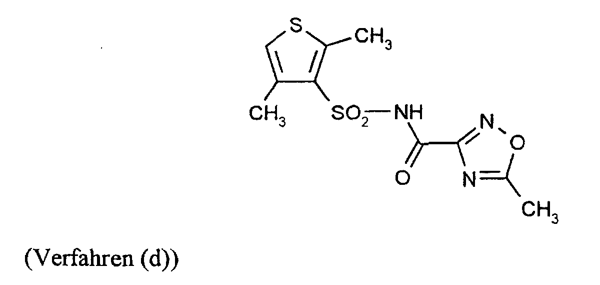

2,0 g (36 mMol) Kaliumhydroxid-Pulver werden bei 20°C bis maximal 35°C zu einer Lösung von 1,52 g (12,0 mMol) 5-Methyl-1,2,4-oxadiazol-3-carboxamid in 150 ml Dioxan gegeben. Nach 30 Minuten werden im Wasserstrahlvakuum bei 30°C bis 35°C ca. 50 ml Dioxan abdestilliert. Anschließend wird die Mischung portionsweise mit 2,65 g (12,6 mMol) 2,4-Dimethyl-thiophen-3-sulfochlorid versetzt und die Reaktionsmischung wird ca. 12 Stunden bei Raumtemperatur (ca. 20°C) gerührt. Anschließend wird im Wasserstrahlvakuum eingeengt, der Rückstand in Wasser aufgenommen und mit 2N-Salzsäure angesäuert. Dann wird zweimal mit je 100 ml Methylenchlorid extrahiert. Die vereinigten organischen Lösungen werden mit Wasser gewaschen, mit Magnesiumsulfat getrocknet und filtriert. Das Filtrat wird im Wasserstrahlvakuum eingeengt und der Rückstand aus Ethanol umkristallisiert.

Man erhält 0,7 g (19% der Theorie) N-(2,4-Dimethyl-thien-3-yl-sulfonyl)-5-methyl-1,2,4-oxadiazol-3-carboxamid vom Schmelzpunkt 164°C.

Analog zu den Beispielen 1 und 2 sowie entsprechend der allgemeinen Beschreibung der erfindungsgemäßen Herstellungsverfahren können beispielsweise auch die in der nachstehenden Tabelle 1 aufgeführten Verbindungen der Formel (I) hergestellt werden.

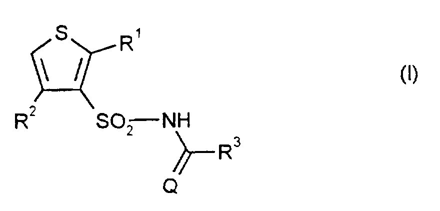

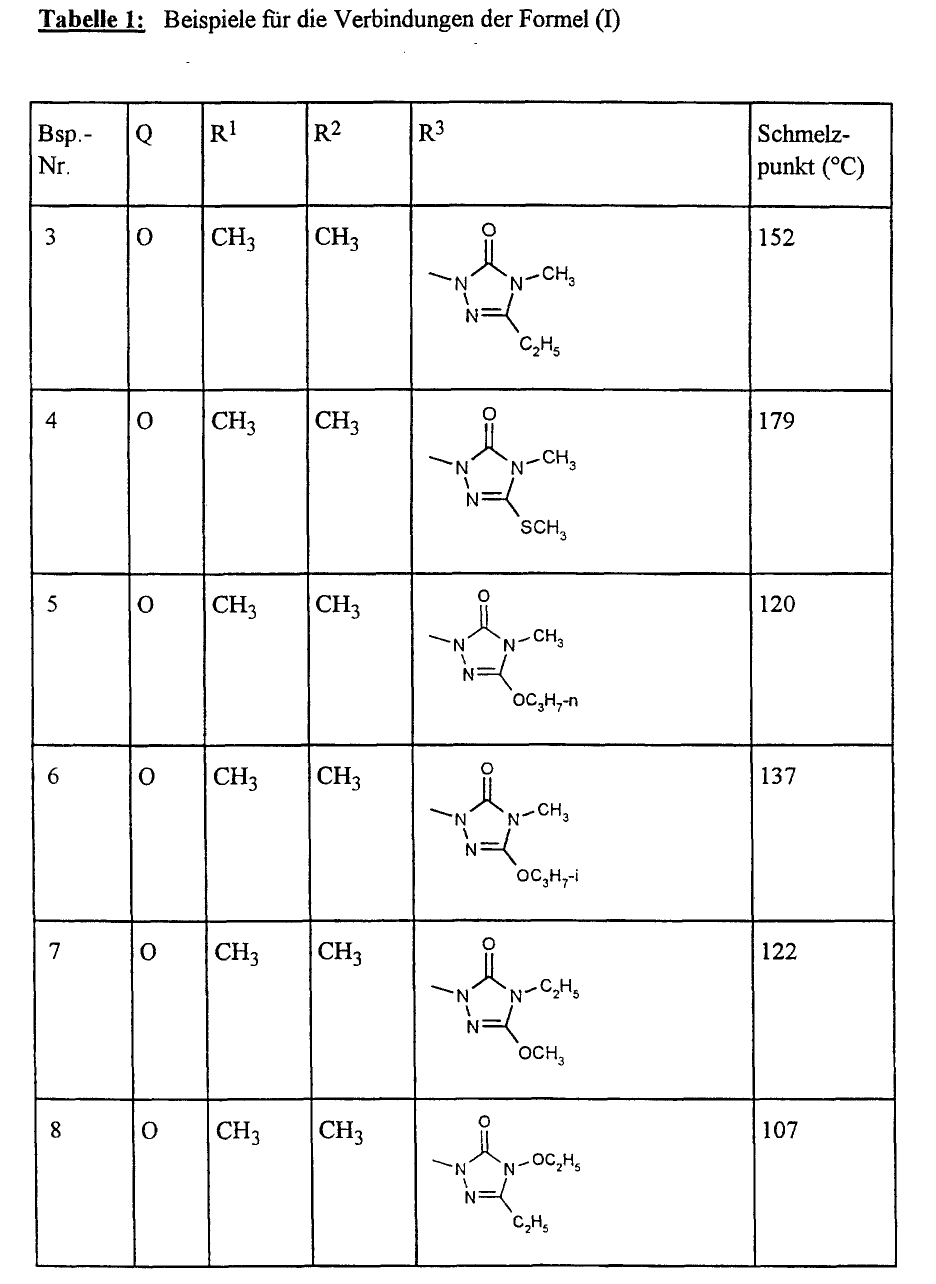

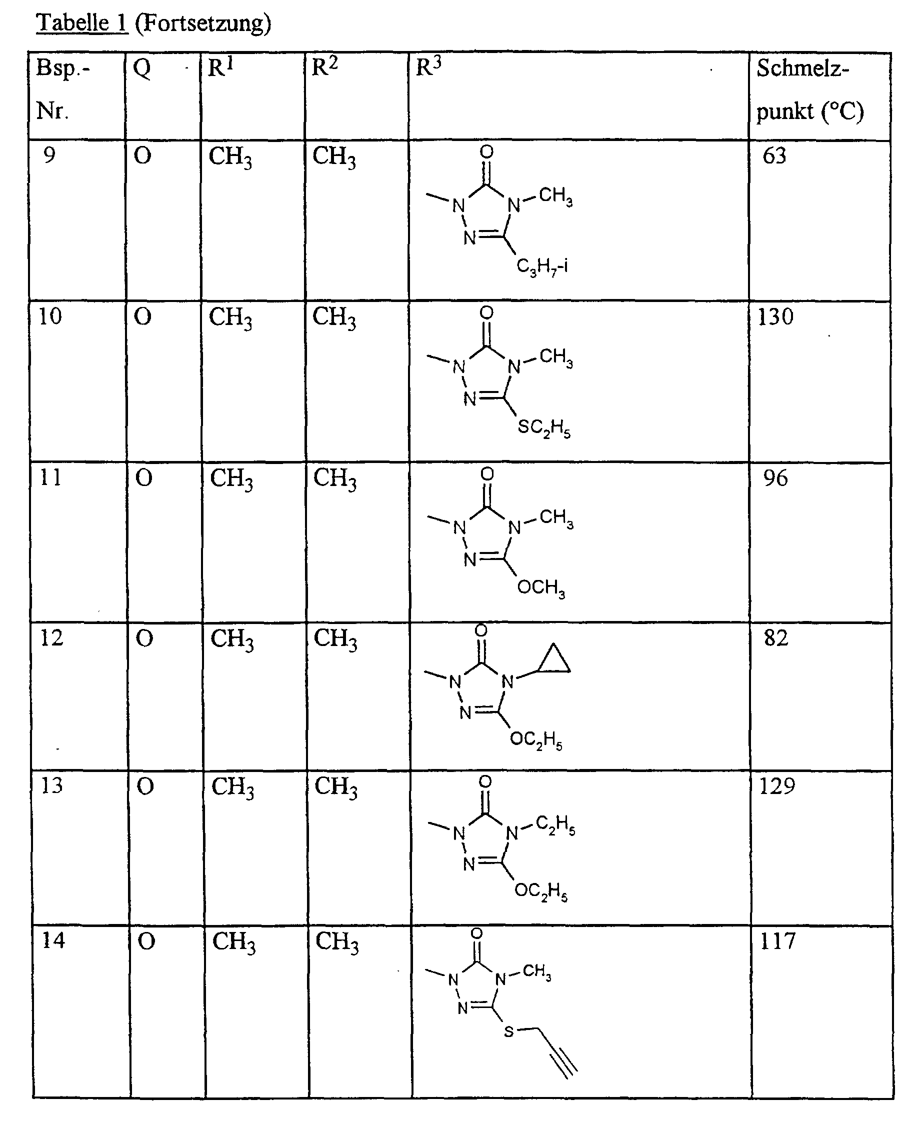

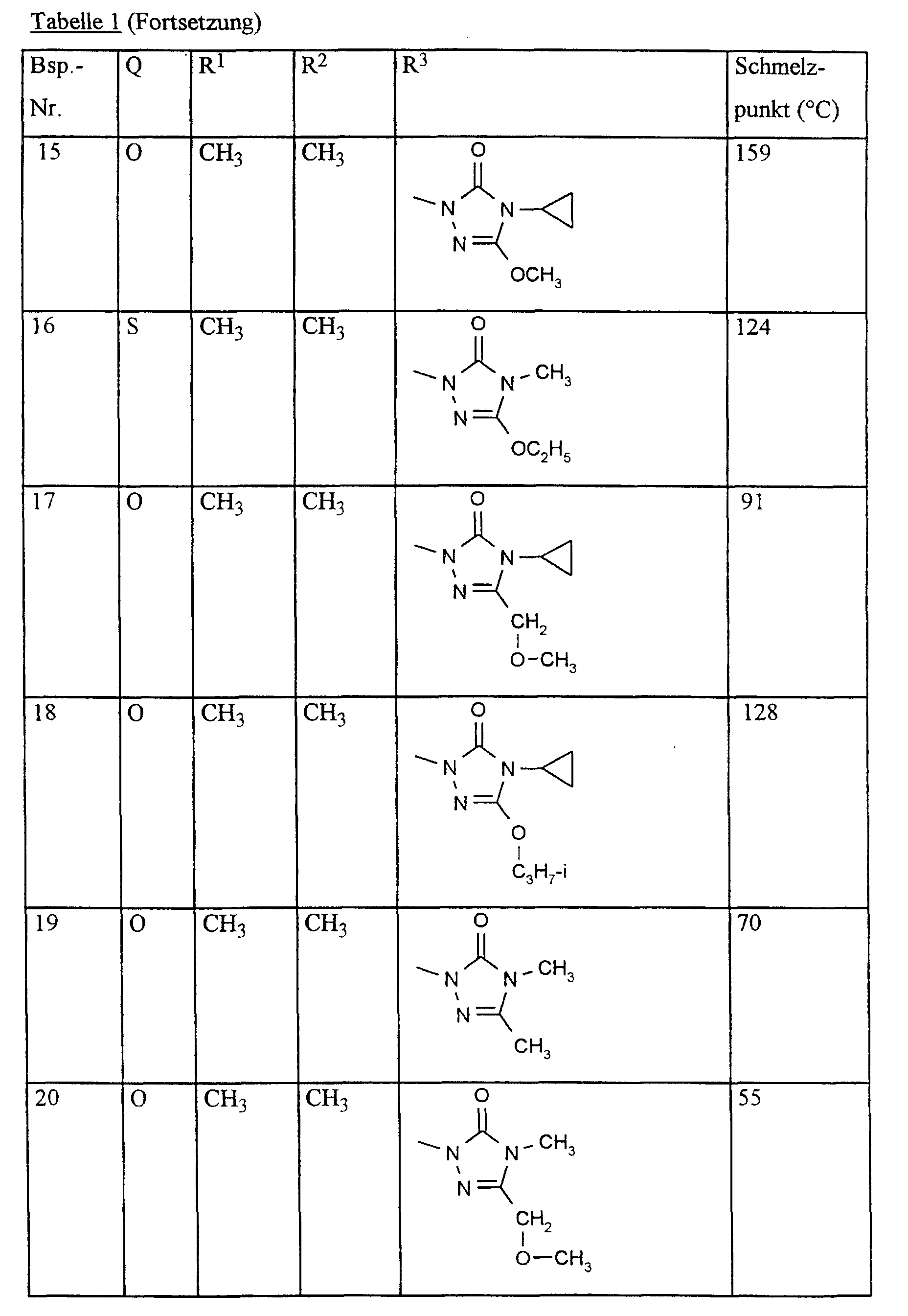

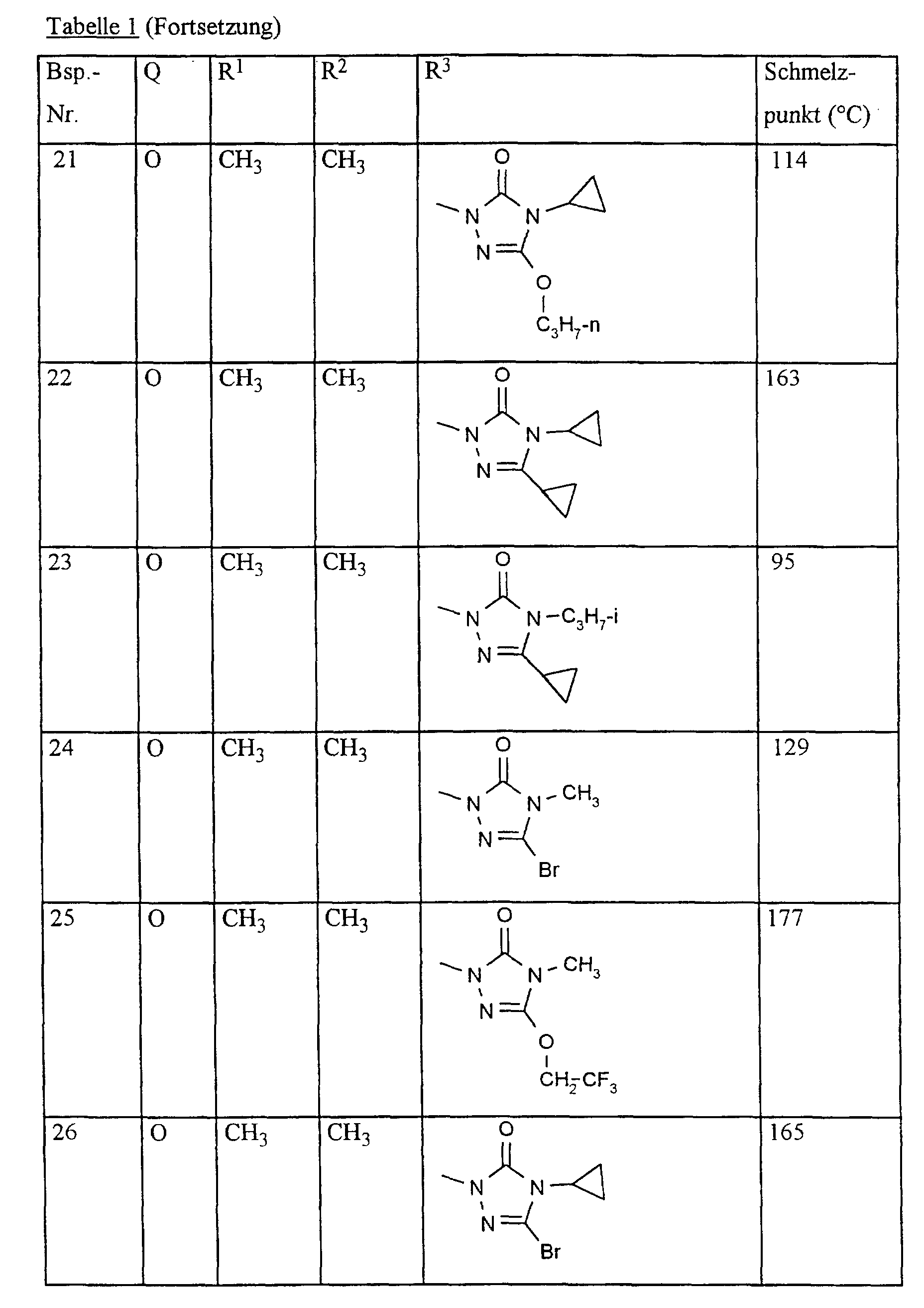

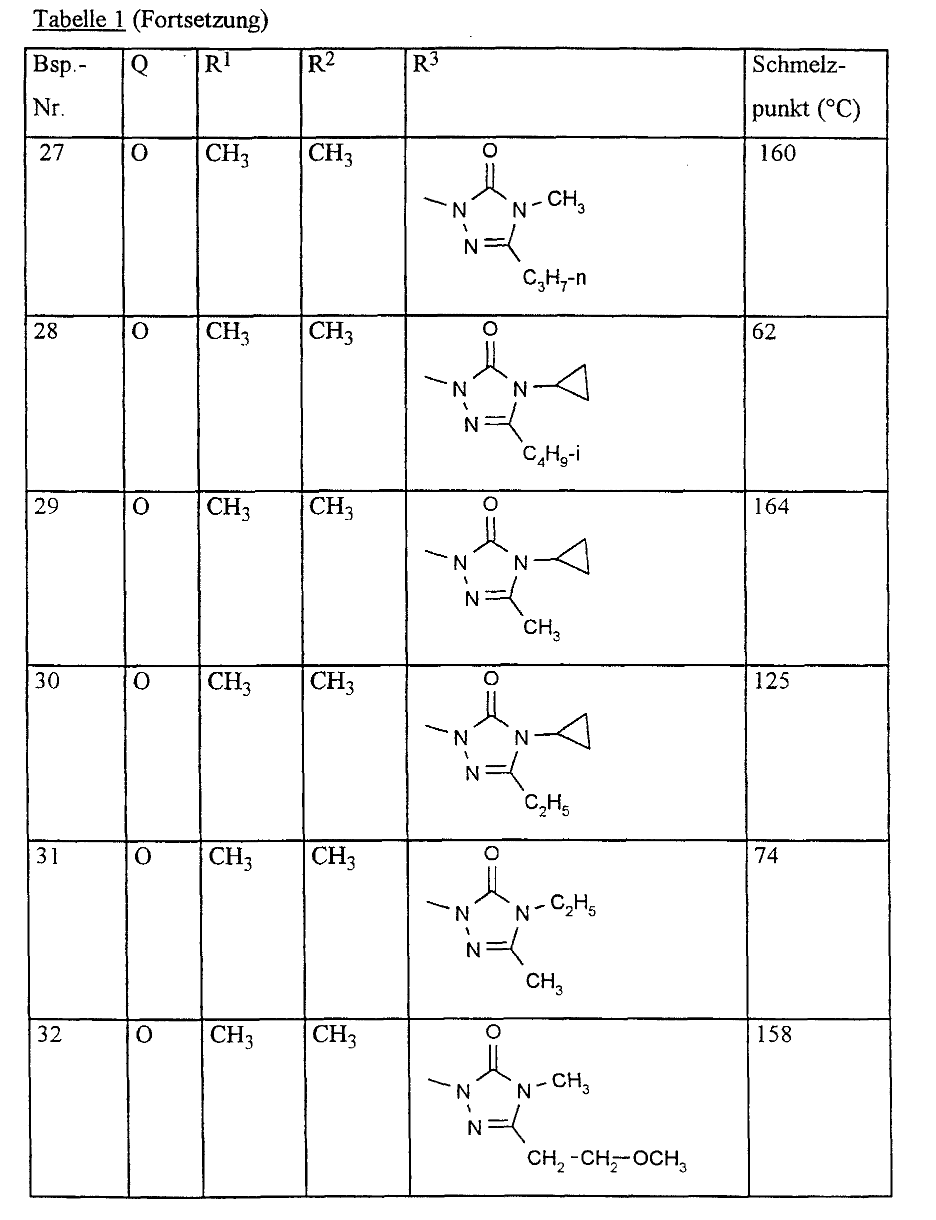

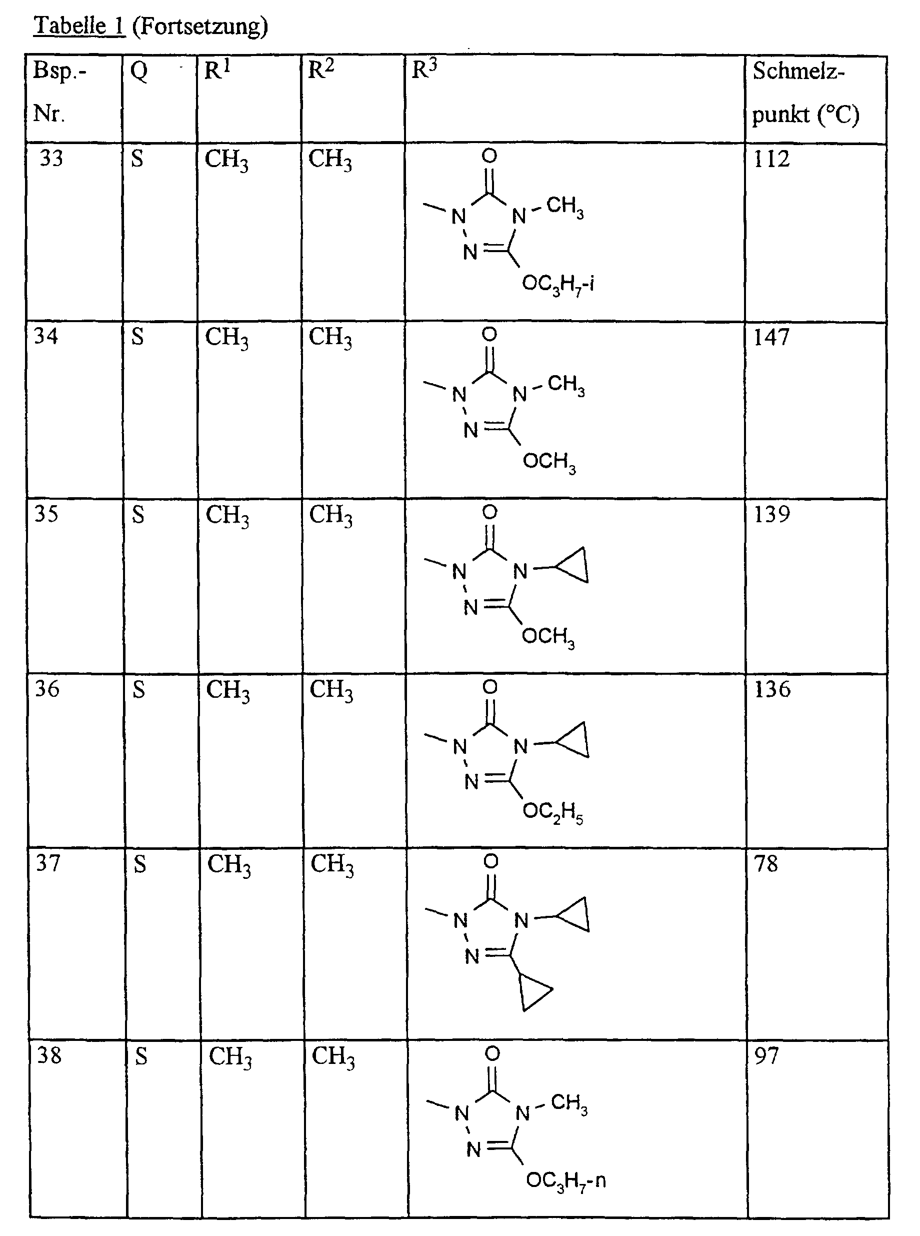

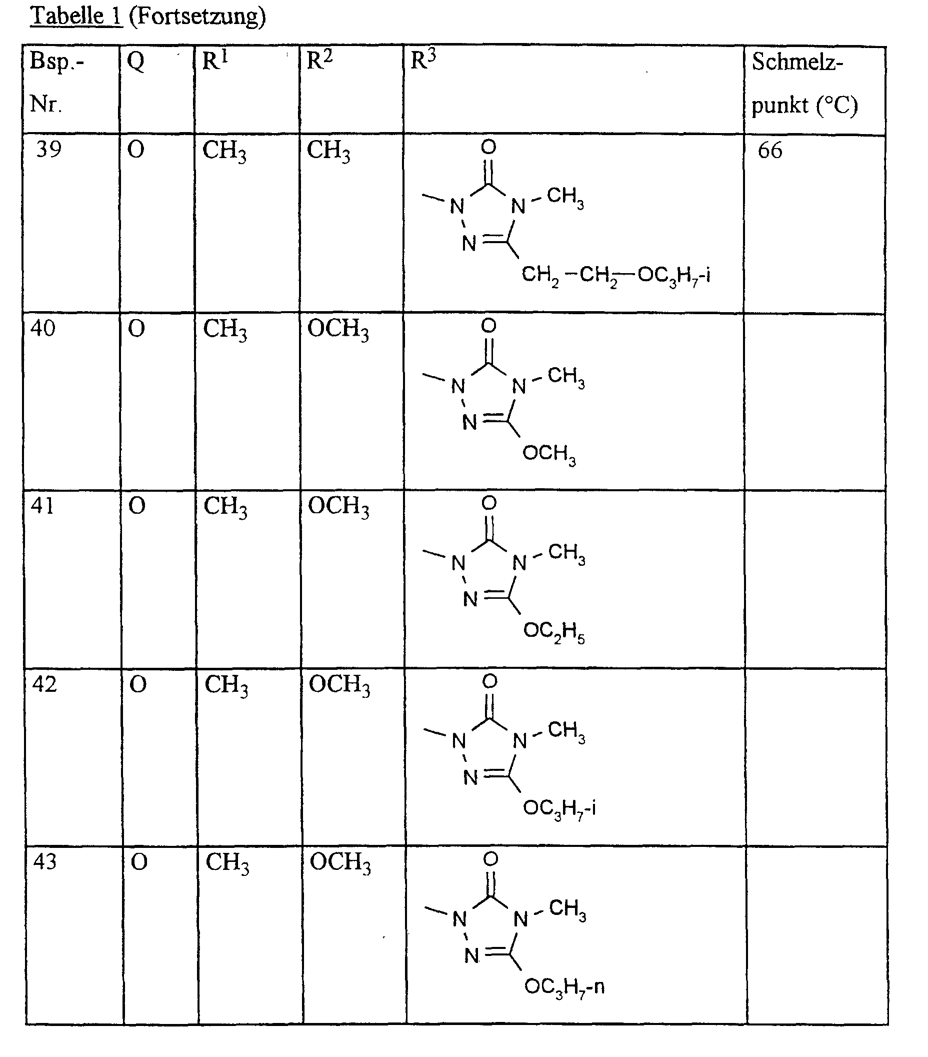

### Ausgangsstoffe der Formel (II):

### Beispiel (II-1)

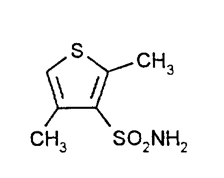

Eine Mischung aus 6,0 g (29 mMol) 2,4-Dimethyl-thiophen-3-sulfochlorid und 30 ml 25%iger wässriger Ammoniaklösung wird 12 Stunden bei Raumtemperatur (ca. 20°C) gerührt. Das hierbei kristallin angefallene Produkt wird dann durch Absaugen isoliert.

Man erhält 4,3 g (80% der Theorie) 2,4-Dimethyl-thiophen-3-sulfonamid vom Schmelzpunkt 135°C.

### Ausgangsstoffe der Formel (IV):

### Beispiel (IV-1)

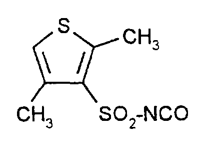

Eine Mischung aus 19,1 g (100 mMol) 2,4-Dimethyl-thiophen-3-sulfonamid, 10,0 g (100 mMol) Butylisocyanat und 100 ml Chloroform wird zum Sieden erhitzt und bei Rückflußtemperatur wird 4 Stunden lang Phosgen in die Mischung geleitet. Anschließend wird im Wasserstrahlvakuum eingeengt und der Rückstand einer Destillation im Ölpumpenvakuum unterworfen.

Man erhält 10,3 g (47% der Theorie) 2,4-Dimethyl-thien-3-yl-sulfonylisocyanat vom Siedebereich 135°C bis 140°C (bei 1 mbar).

### Ausgangsstoffe der Formel (VI):

### Beispiel (VI-1)

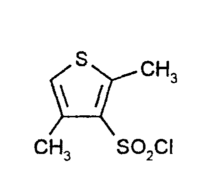

Eine Lösung von 13,9 g (109 mMol) 3-Amino-2,4-dimethyl-thiophen in 30 ml 10%iger Salzsäure wird auf 0°C abgekühlt und mit 50 ml konz. Salzsäure versetzt. Unter Kühlen auf 0°C bis -5°C wird dann unter Rühren eine Lösung von 8,6 g (125 mMol) Natriumnitrit in 22 ml Wasser tropfenweise dazu gegeben. Die Reaktionsmischung wird etwa eine Stunde lang bei 0°C bis -5°C gerührt. Anschließend wird überschüssiges Natriumnitrit mit Amidosulfonsäure zerstört. Die so erhaltene Diazoniumsalzlösung wird bei ca. 15°C zu einer Lösung von 12 g Schwefeldioxid in 100 ml 1,2-Dichlor-ethan tropfenweise gegeben. Dann werden 600 mg Kupfer(I)-chlorid und 600 mg Dodecyl-trimethylammoniumbromid dazu gegeben und die Reaktionsmischung wird etwa eine Stunde lang bei ca. 40°C und weitere 12 Stunden bei Raumtemperatur (ca. 20°C) gerührt. Nach Zugabe von 6 g 30%iger Hydrogenperoxid-Lösung wird die Mischung weitere 30 Minuten gerührt. Die organische Phase wird dann abgetrennt, zweimal mit Wasser gewaschen, mit Magnesiumsulfat getrocknet und filtriert. Das Filtrat wird im Wasserstrahlvakuum eingeengt, der Rückstand mit Petrolether digeriert und das kristallin angefallene Produkt durch Absaugen isoliert.

Man erhält 9,6 g (42% der Theorie) 2,4-Dimethyl-thiophen-3-sulfochlorid vom Schmelzpunkt 79°C.

Jeweils analog zu den Beispielen (II-1), (IV-1) und (VI-1) können beispielsweise auch die in der nachfolgenden Tabelle 2 aufgeführten Verbindungen der Formeln (II), (IV) und (VI) hergestellt werden:

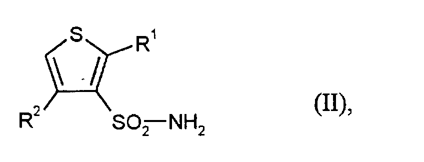

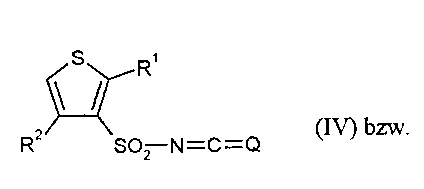

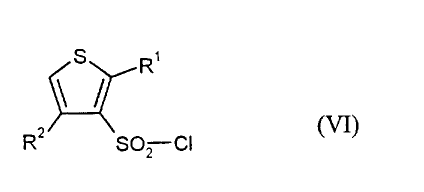

**Tabelle 2:**

| Beispiele für die Verbindungen der Formeln (II), (IV) und (VI) [d.h. die Reste R¹ und R² gelten für jede dieser 3 Formeln]; Q ist O oder S. | | |
|---|---|---|
| Beispiel-Nr. II- IV- VI- | R¹ | R² |
| 2 | CH₃ | C₂H₅ |
| 3 | CH₃ | C₃H₇-n |
| 4 | CH₃ | C₃H₇-i |
| 5 | CH₃ | CF₃ |
| 6 | CH₃ | Cl |
| 7 | CH₃ | OCH₃ |
| 8 | CH₃ | OC₂H₅ |
| 9 | CH₃ | OC₃H₇-n |
| 10 | CH₃ | OC₃H₇-i |
| 11 | C₂H₅ | CH₃ |
| 12 | C₃H₇-n | CH₃ |
| 13 | C₃H₇-i | CH₃ |

### Anwendungsbeispiele:

### Beispiel A

### Pre-emergence-Test

| | |
|---|---|
| Lösungsmittel | 5 Gewichtsteile Aceton |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät. Nach ca. 24 Stunden wird der Boden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit.

Nach drei Wochen wird der Schädigungsgrad der Pflanzen bonitiert in % Schädigung im Vergleich zur Entwicklung der unbehandelten Kontrolle. Es bedeuten:
- 0% =: keine Wirkung (wie unbehandelte Kontrolle)
- 100% =: totale Vernichtung

In diesem Test zeigen beispielsweise die Verbindungen gemäß Herstellungsbeispiel 1, 3, 4, 5, 11 und 12 bei teilweise guter Verträglichkeit gegenüber Kulturpflanzen, wie z.B. Mais und Weizen, sehr starke Wirkung gegen Unkräuter (vgl. Tabelle A).
"ai." (active ingredient) = Wirkstoff

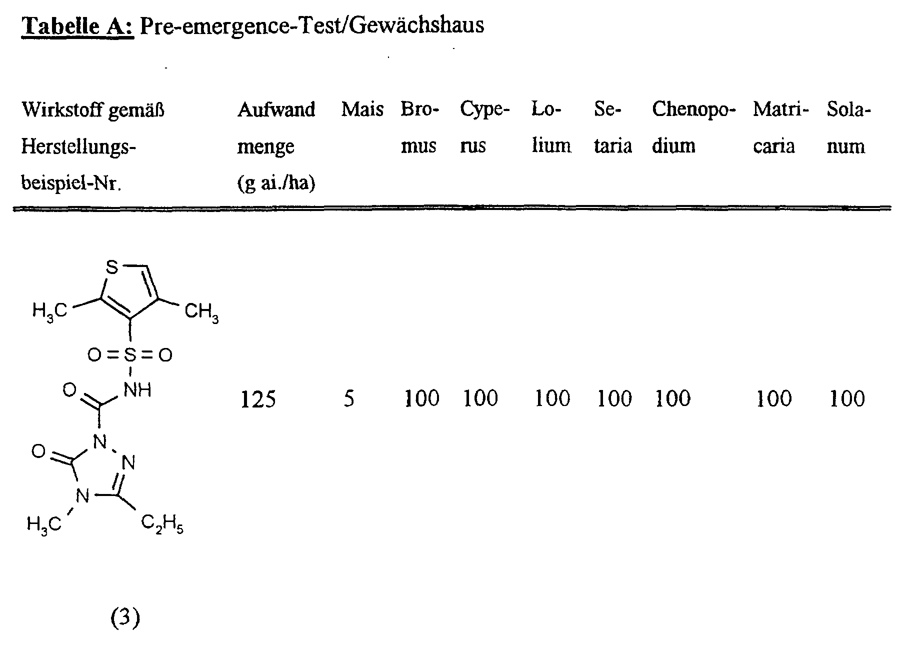

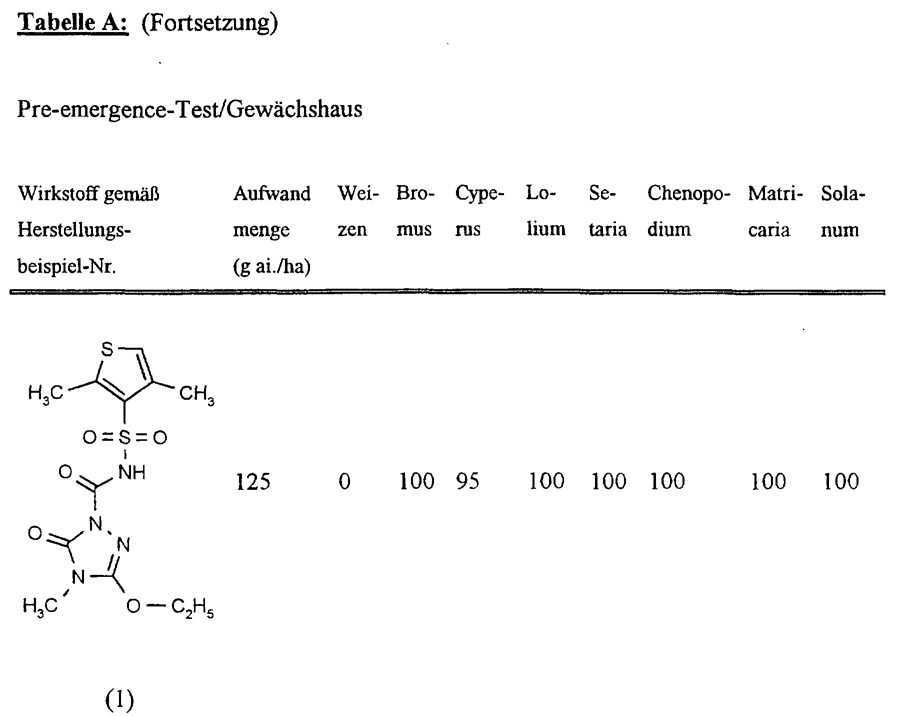

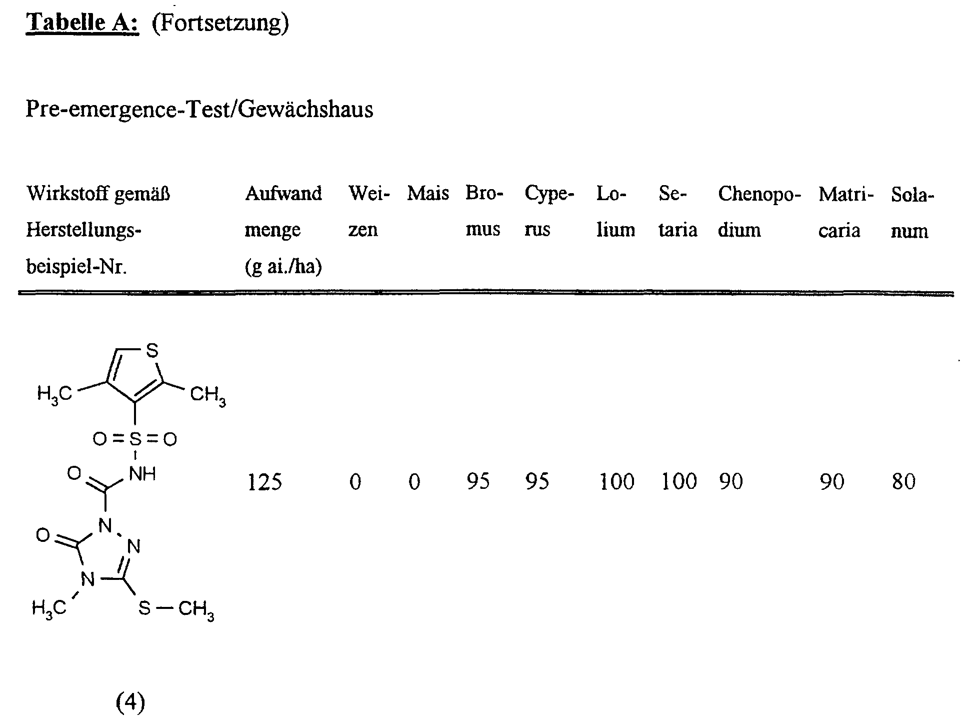

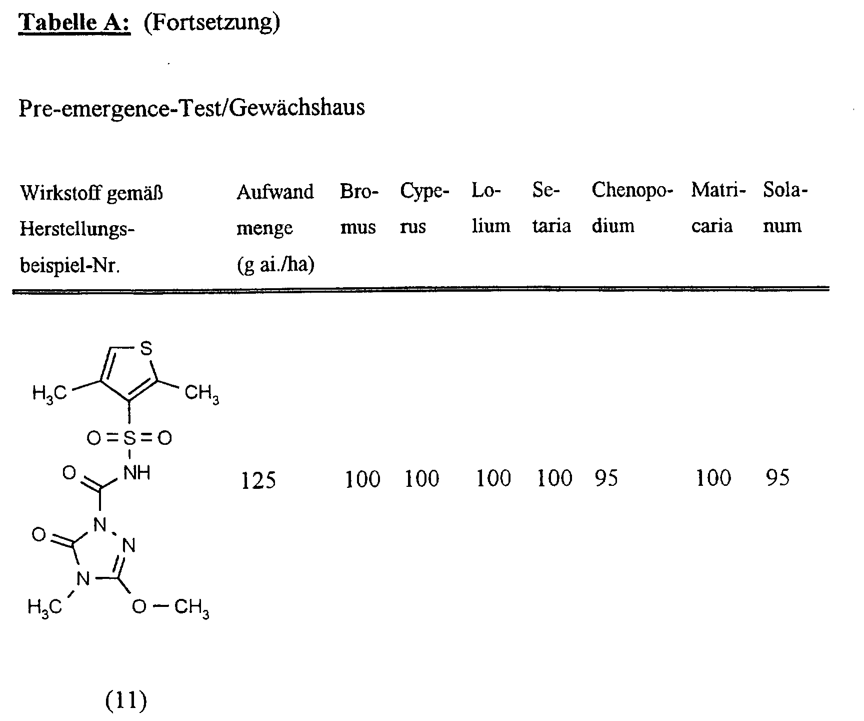

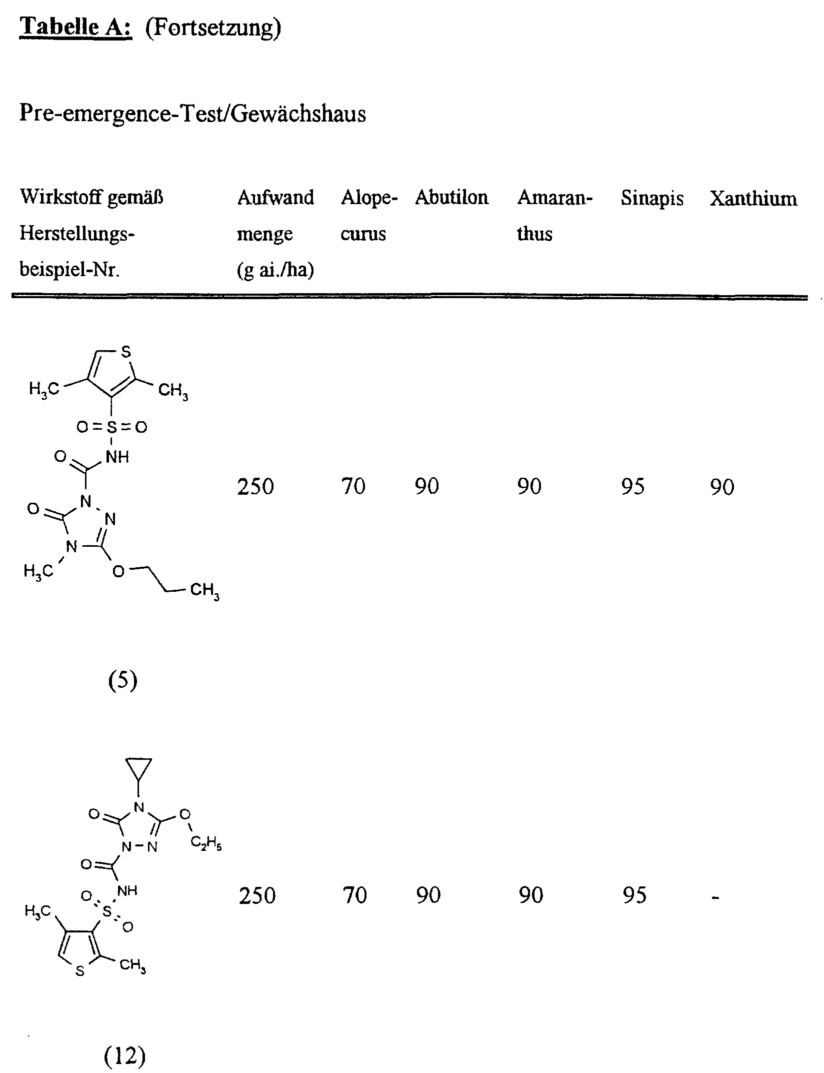

### Beispiel B

### Post-emergence-Test

| | |
|---|---|
| Lösungsmittel | 5 Gewichtsteile Aceton |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5-15 cm haben so, daß die jeweils gewünschten Wirkstoffmengen pro Flächeneinheit ausgebracht werden. Die Konzentration der Spritzbrühe wird so gewählt, daß in 1000 1 Wasser/ha die jeweils gewünschten Wirkstoffmengen ausgebracht werden.

Nach drei Wochen wird der Schädigungsgrad der Pflanzen bonitiert in % Schädigung im Vergleich zur Entwicklung der unbehandelten Kontrolle.

Es bedeuten:
- 0 % =: keine Wirkung (wie unbehandelte Kontrolle)
- 100 % =: totale Vernichtung

In diesem Test zeigen beispielsweise die Verbindungen gemäß Herstellungsbeispiel 6, 13 und 14 bei teilweise guter Verträglichkeit gegenüber Kulturpflanzen, wie z.B. Mais, sehr starke Wirkung gegen Unkräuter (vgl. Tabelle B).

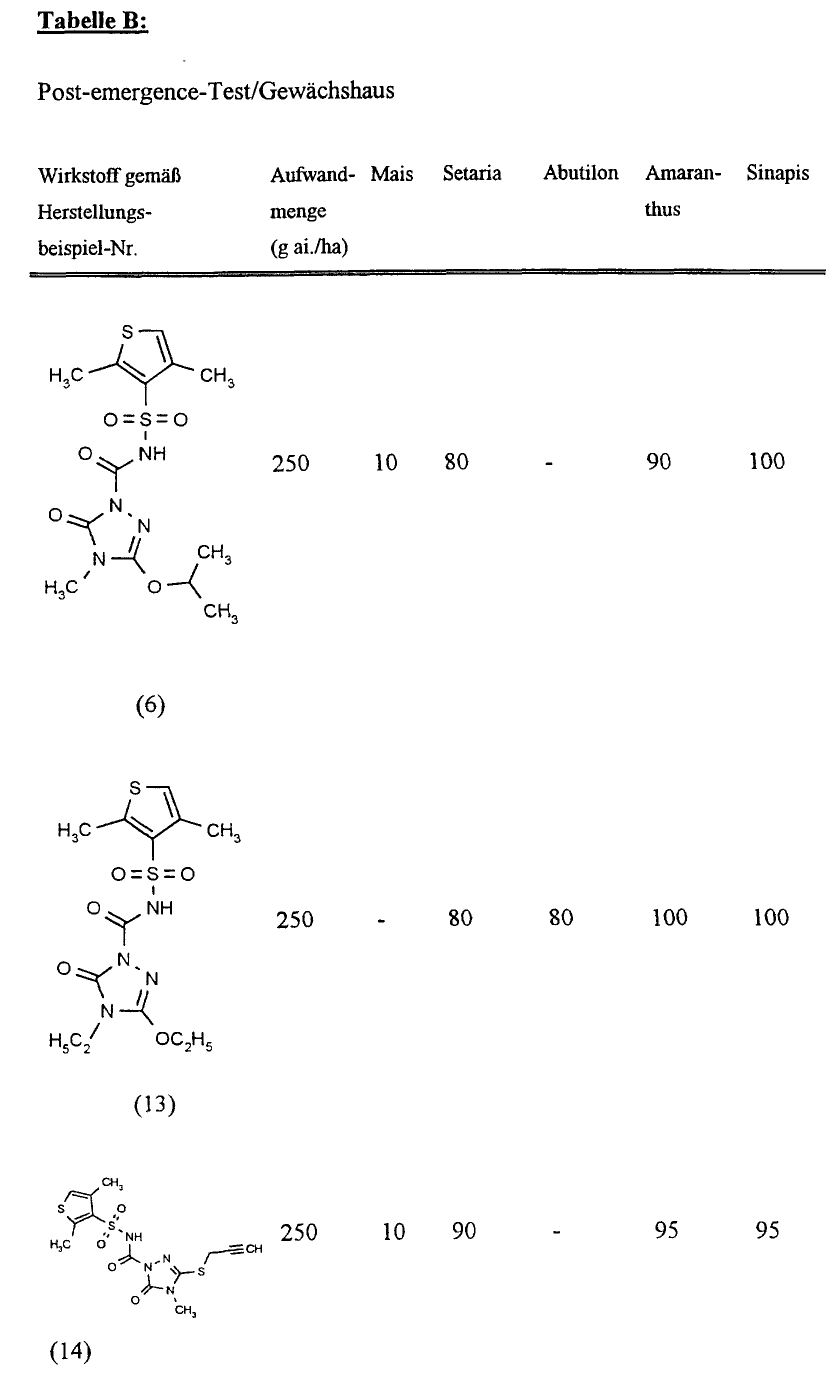

## Patentansprüche

1. Thienylsulfonylamino(thio)carbonylverbindungen der allgemeinen Formel (I) 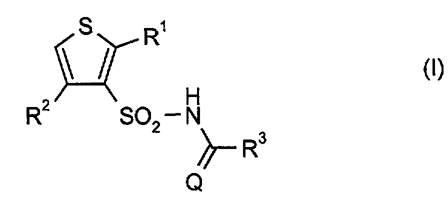 in welcher
Q für Sauerstoff oder Schwefel steht,
R¹ für Cyano, Halogen oder jeweils gegebenenfalls durch Cyano, Halogen oder C₁-C₄-Alkoxy substituiertes Alkyl, Alkenyl, Alkinyl, Alkoxy, Alkenyloxy oder Alkinyloxy mit jeweils bis zu 6 Kohlenstoffatomen steht,
R² für Cyano, Halogen oder jeweils gegebenenfalls durch Cyano, Halogen oder C₁-C₄-Alkoxy substituiertes Alkyl, Alkenyl, Alkinyl, Alkoxy, Alkenyloxy oder Alkinyloxy mit jeweils bis zu 6 Kohlenstoffatomen steht, und
R³ für jeweils gegebenenfalls substituiertes Heterocyclyl der nachstehenden Formeln steht 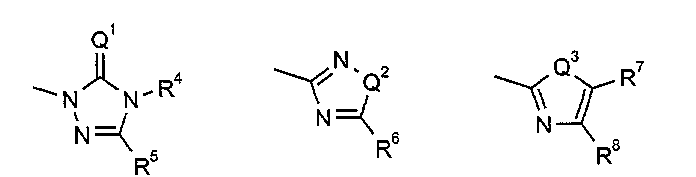
worin
Q¹, Q² und Q³ jeweils für Sauerstoff oder Schwefel stehen sowie
R⁴ für Wasserstoff, Hydroxy, Amino, Cyano, für C₂-C₁₀-Alkylidenamino, für gegebenenfalls durch Fluor, Chlor, Brom, Cyano, C₁-C₄-Alkoxy, C₁-C₄-Alkyl-carbonyl oder C₁-C₄-Alkoxy-carbonyl substituiertes C₁-C₆-Alkyl, für jeweils gegebenenfalls durch Fluor, Chlor und/oder Brom substituiertes C₂-C₆-Alkenyl oder C₂-C₆-Alkinyl, für jeweils gegebenenfalls durch Fluor, Chlor, Brom, Cyano, C₁-C₄-Alkoxy oder C₁-C₄-Alkoxy-carbonyl substituiertes C₁-C₆-Alkoxy, C₁-C₆-Alkylamino oder C₁-C₆-Alkyl-carbonylamino, für C₃-C₆-Alkenyloxy, für Di-(C₁-C₄-alkyl)-amino, für jeweils gegebenenfalls durch Fluor, Chlor, Brom, Cyano und/oder C₁-C₄-Alkyl substituiertes C₃-C₆-Cycloalkyl, C₃-C₆-Cycloalkylamino oder C₃-C₆-Cycloalkyl-C₁-C₄-alkyl, oder für jeweils gegebenenfalls durch Fluor, Chlor, Brom, Cyano, Nitro, C₁-C₄-Alkyl, Trifluormethyl und/oder C₁-C₄-Alkoxy substituiertes Phenyl oder Phenyl-C₁-C₄-alkyl steht,
R⁵ für Wasserstoff, Hydroxy, Mercapto, Amino, Cyano, Fluor, Chlor, Brom, Iod, für gegebenenfalls durch Fluor, Chlor, Brom, Cyano, C₁-C₄-Alkoxy, C₁-C₄-Alkyl-carbonyl oder C₁-C₄-Alkoxy-carbonyl substituiertes C₁-C₆-Alkyl, für jeweils gegebenenfalls durch Fluor, Chlor und/oder Brom substituiertes C₂-C₆-Alkenyl oder C₂-C₆-Alkinyl, für jeweils gegebenenfalls durch Fluor, Chlor, Cyano, C₁-C₄-Alkoxy oder C₁-C₄-Alkoxy-carbonyl substituiertes C₁-C₆-Alkoxy, C₁-C₆-Alkylthio, C₁-C₆-Alkylamino oder C₁-C₆-Alkyl-carbonylamino, für C₃-C₆-Alkenyloxy, C₃-C₆-Alkinyloxy, C₃-C₆-Alkenylthio, C₃-C₆-Alkinylthio, C₃-C₆-Alkenylamino oder C₃-C₆-Alkinylamino, für Di-(C₁-C₄-alkyl)-amino, für jeweils gegebenenfalls durch Methyl und/oder Ethyl substituiertes Aziridino, Pyrrolidino, Piperidino oder Morpholino, für jeweils gegebenenfalls durch Fluor, Chlor, Brom, Cyano und/oder C₁-C₄-Alkyl substituiertes C₃-C₆-Cycloalkyl, C₅-C₆-Cycloalkenyl, C₃-C₆-Cycloalkyloxy, C₃-C₆-Cycloalkylthio, C₃-C₆-Cycloalkylamino, C₃-C₆-Cycloalkyl-C₁-C₄-alkyl, C₃-C₆-Cycloalkyl-C₁-C₄-alkoxy, C₃-C₆-Cycloalkyl-C₁-C₄-alkylthio oder C₃-C₆-Cycloalkyl-C₁-C₄-alkylamino, oder für jeweils gegebenenfalls durch Fluor, Chlor, Brom, Cyano, Nitro, C₁-C₄-Alkyl, Trifluormethyl, C₁-C₄-Alkoxy und/oder C₁-C₄-Alkoxy-carbonyl substituiertes Phenyl, Phenyl-C₁-C₄-alkyl, Phenoxy, Phenyl-C₁-C₄-alkoxy, Phenylthio, Phenyl-C₁-C₄-alkylthio, Phenylamino oder Phenyl-C₁-C₄-alkylamino steht, oder
R⁴ und R⁵ zusammen für gegebenenfalls verzweigtes Alkandiyl mit 3 bis 11 Kohlenstoffatomen stehen, ferner
R⁶, R⁷ und R⁸ gleich oder verschieden sind und für Wasserstoff, Cyano, Fluor, Chlor, Brom, oder für jeweils gegebenenfalls durch Fluor, Chlor, Brom oder C₁-C₄-Alkoxy substituiertes Alkyl, Alkenyl, Alkinyl, Alkoxy, Alkenyloxy, Alkinyloxy, Alkylthio, Alkenylthio, Alkinylthio, Alkylsulfinyl oder Alkylsulfonyl mit jeweils bis zu 6 Kohlenstoffatomen oder für gegebenenfalls durch Cyano, Fluor, Chlor, Brom oder C₁-C₄-Alkyl substituiertes Cycloalkyl mit 3 bis 6 Kohlenstoffatomen stehen,
sowie Salze von Verbindungen der Formel (I).

2. Verbindungen der Formel (I) gemäß Anspruch 1, **dadurch gekennzeichnet, dass** darin
Q für Sauerstoff oder Schwefel steht,
R¹ für Cyano, Halogen, für gegebenenfalls durch Cyano, Halogen oder C₁-C₄-Alkoxy substituiertes C₁-C₄-Alkyl, für jeweils gegebenenfalls durch Cyano oder Halogen substituiertes C₂-C₄-Alkenyl oder C₂-C₄-Alkinyl, für gegebenenfalls durch Cyano, Halogen oder C₁-C₄-Alkoxy substituiertes C₁-C₄-Alkoxy, oder für jeweils gegebenenfalls durch Cyano oder Halogen substituiertes C₂-C₄-Alkenyloxy oder C₂-C₄-Alkinyloxy steht,
R² für Cyano, Halogen, für gegebenenfalls durch Cyano, Halogen oder C₁-C₄-Alkoxy substituiertes C₁-C₄-Alkyl, für jeweils gegebenenfalls durch Cyano oder Halogen substituiertes C₂-C₄-Alkenyl oder C₂-C₄-Alkinyl, für gegebenenfalls durch Cyano, Halogen oder C₁-C₄-Alkoxy substituiertes C₁-C₄-Alkoxy, oder für jeweils gegebenenfalls durch Cyano oder Halogen substituiertes C₂-C₄-Alkenyloxy oder C₂-C₄-Alkinyloxy steht, und
R³ für jeweils gegebenenfalls substituiertes Heterocyclyl der nachstehenden Formeln steht 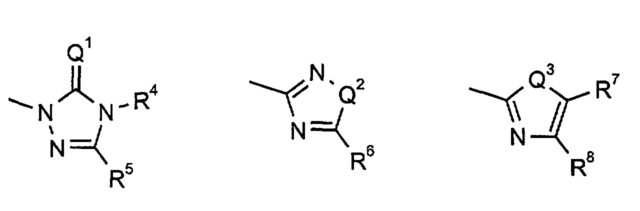
worin
Q¹, Q² und Q³ jeweils für Sauerstoff oder Schwefel stehen sowie
R⁴ für Wasserstoff, Hydroxy, Amino, Cyano, für C₂-C₁₀-Alkylidenamino, für gegebenenfalls durch Fluor, Chlor, Brom, Cyano, C₁-C₄-Alkoxy, C₁-C₄-Alkyl-carbonyl oder C₁-C₄-Alkoxy-carbonyl substituiertes C₁-C₆-Alkyl, für jeweils gegebenenfalls durch Fluor, Chlor und/oder Brom substituiertes C₂-C₆-Alkenyl oder C₂-C₆-Alkinyl, für jeweils gegebenenfalls durch Fluor, Chlor, Brom, Cyano, C₁-C₄-Alkoxy oder C₁-C₄-Alkoxy-carbonyl substituiertes C₁-C₆-Alkoxy, C₁-C₆-Alkylamino oder C₁-C₆-Alkyl-carbonylamino, für C₃-C₆-Alkenyloxy, für Di-(C₁-C₄-alkyl)-amino, für jeweils gegebenenfalls durch Fluor, Chlor, Brom, Cyano und/oder C₁-C₄-Alkyl substituiertes C₃-C₆-Cycloalkyl, C₃-C₆-Cycloalkylamino oder C₃-C₆-Cycloalkyl-C₁-C₄-alkyl, oder für jeweils gegebenenfalls durch Fluor, Chlor, Brom, Cyano, Nitro, C₁-C₄-Alkyl, Trifluormethyl und/oder C₁-C₄-Alkoxy substituiertes Phenyl oder Phenyl-C₁-C₄-alkyl steht,
R⁵ für Wasserstoff, Hydroxy, Mercapto, Amino, Cyano, Fluor, Chlor, Brom, Iod, für gegebenenfalls durch Fluor, Chlor, Brom, Cyano, C₁-C₄-Alkoxy, C₁-C₄-Alkyl-carbonyl oder C₁-C₄-Alkoxy-carbonyl substituiertes C₁-C₆-Alkyl, für jeweils gegebenenfalls durch Fluor, Chlor und/oder Brom substituiertes C₂-C₆-Alkenyl oder C₂-C₆-Alkinyl, für jeweils gegebenenfalls durch Fluor, Chlor, Cyano, C₁-C₄-Alkoxy oder C₁-C₄-Alkoxy-carbonyl substituiertes C₁-C₆-Alkoxy, C₁-C₆-Alkylthio, C₁-C₆-Alkylamino oder C₁-C₆-Alkyl-carbonylamino, für C₃-C₆-Alkenyloxy, C₃-C₆-Alkinyloxy, C₃-C₆-Alkenylthio, C₃-C₆-Alkinylthio, C₃-C₆-Alkenylamino oder C₃-C₆-Alkinylamino, für Di-(C₁-C₄-alkyl)-amino, für jeweils gegebenenfalls durch Methyl und/oder Ethyl substituiertes Aziridino, Pyrrolidino, Piperidino oder Morpholino, für jeweils gegebenenfalls durch Fluor, Chlor, Brom, Cyano und/oder C₁-C₄-Alkyl substituiertes C₃-C₆-Cycloalkyl, C₅-C₆-Cycloalkenyl, C₃-C₆-Cycloalkyloxy, C₃-C₆-Cycloalkylthio, C₃-C₆-Cycloalkylamino, C₃-C₆-Cycloalkyl-C₁-C₄-alkyl, C₃-C₆-Cycloalkyl-C₁-C₄-alkoxy, C₃-C₆-Cycloalkyl-C₁-C₄-alkylthio oder C₃-C₆-Cycloalkyl-C₁-C₄-alkylamino, oder für jeweils gegebenenfalls durch Fluor, Chlor, Brom, Cyano, Nitro, C₁-C₄-Alkyl, Trifluormethyl, C₁-C₄-Alkoxy und/oder C₁-C₄-Alkoxy-carbonyl substituiertes Phenyl, Phenyl-C₁-C₄-alkyl, Phenoxy, Phenyl-C₁-C₄-alkoxy, Phenylthio, Phenyl-C₁-C₄-alkylthio, Phenylamino oder Phenyl-C₁-C₄-alkylamino steht, oder
R⁴ und R⁵ zusammen für gegebenenfalls verzweigtes Alkandiyl mit 3 bis 11 Kohlenstoffatomen stehen, ferner
R⁶, R⁷ und R⁸ gleich oder verschieden sind und für Wasserstoff, Cyano, Fluor, Chlor, Brom, oder für jeweils gegebenenfalls durch Fluor, Chlor, Brom oder C₁-C₄-Alkoxy substituiertes Alkyl, Alkenyl, Alkinyl, Alkoxy, Alkenyloxy, Alkinyloxy, Alkylthio, Alkenylthio, Alkinylthio, Alkylsulfinyl oder Alkylsulfonyl mit jeweils bis zu 6 Kohlenstoffatomen oder für gegebenenfalls durch Cyano, Fluor, Chlor, Brom oder Ci-C4-Alkyl substituiertes Cycloalkyl mit 3 bis 6 Kohlenstoffatomen stehen,
sowie die Natrium-, Kalium-, Magnesium-, Calcium-, Ammonium-, C₁-C₄-Alkyl-ammonium-, Di-(C₁-C₄-alkyl)-ammonium-, Tri-(C₁-C₄-alkyl)-ammonium-, Tetra-(C₁-C₄-alkyl)-ammonium-, Tri-(C₁-C₄-alkyl)-sulfonium-, C₅-oder C₆-Cycloalkyl-ammonium- und Di-(C₁-C₂-alkyl)-benzyl-ammonium-Salze von Verbindungen der Formel (I).

3. Verfahren zur Herstellung von Thienylsulfonylamino(thio)carbonylverbindungen der allgemeinen Formel (I) gemäß Anspruch 1, dadurch gekenzeichnet, dass man
(a) Sulfonamide der allgemeinen Formel (II), 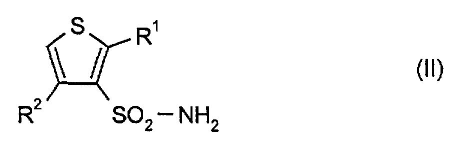 in welcher
R¹ and R² die in Anspruch 1 angegebene Bedeutung haben,
mit (Thio)Carbonsäurederivaten der allgemeinen Formel (III), 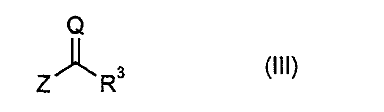 in welcher
Q und R³ die in Anspruch 1 angegebene Bedeutung haben und
Z für Halogen, Alkoxy, Aryloxy oder Arylalkoxy steht,
gegebenenfalls in Gegenwart eines Säureakzeptors und gegebenenfalls in Gegenwart eines Verdünnungsmittels umsetzt,
oder dass man
(b) Sulfonyliso(thio)cyanate der allgemeinen Formel (IV) 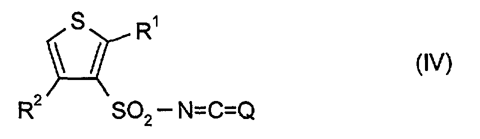 in welcher
Q, R¹ und R² die oben angegebene Bedeutung haben,
mit Heterocyclen der allgemeinen Formel (V)
H-R³⁻¹ (V)
in welcher
R³⁻¹ für gegebenenfalls substituiertes Triazolinyl der nachstehenden Formel 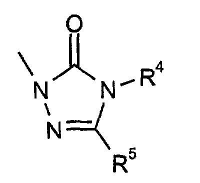 worin Q¹ für Sauerstoff oder Schwefel steht und die Reste R⁴ und R⁵ die oben angegebene Bedeutung haben,
gegebenenfalls in Gegenwart eines Reaktionshilfsmittels und gegebenenfalls in Gegenwart eines Verdünnungsmittels umsetzt,
oder dass man
(c) Sulfonsäurechloride der allgemeinen Formel (VI) 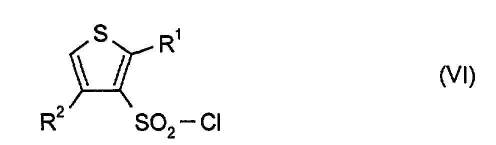 in welcher
R¹ und R² die oben angegebene Bedeutung haben,
mit Heterocyclen der allgemeinen Formel (V)
H-R³⁻¹ (V)
in welcher
R³⁻¹ die oben bei (b)angegebene Bedeutung hat,
und Metall(thio)cyanaten der allgemeinen Formel (VII)
MQCN (VII)
in welcher
Q die oben angegebene Bedeutung hat und
M für ein Metalläquivalent steht,
gegebenenfalls in Gegenwart eines Reaktionshilfsmittels und gegebenenfalls in Gegenwart eines Verdünnungsmittels umsetzt,
oder dass man
(d) Sulfonsäurechloride der allgemeinen Formel (VI) 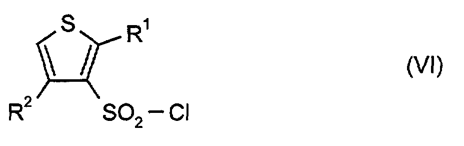 in welcher
R¹ und R² die oben angegebene Bedeutung haben,
mit (Thio)Carbonsäureamiden der allgemeinen Formel (VIII) 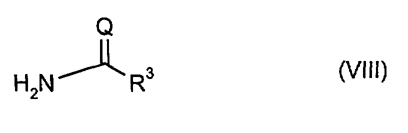 in welcher
Q und R3 die oben bei Formel (I) in Anspruch 1 angegebene Bedeutung haben,
gegebenenfalls in Gegenwart eines Säureakzeptors und gegebenenfalls in Gegenwart eines Verdünnungsmittels umsetzt,
oder dass man
(e) Sulfonylamino(thio)carbonylverbindungen der allgemeinen Formel (IX) 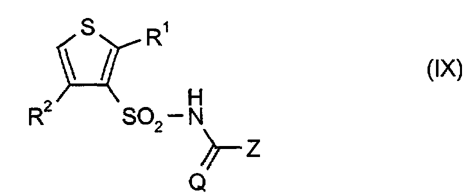
in welcher
Q, R¹ und R² die oben angegebene Bedeutung haben und
Z für Halogen, Alkoxy, Aryloxy oder Arylalkoxy steht,
mit Heterocyclen der allgemeinen Formel (V)
H-R³⁻¹ (V)
in welcher
R³⁻¹ die oben bei (b) angegebene Bedeutung hat,
gegebenenfalls in Gegenwart eines Säureakzeptors und gegebenenfalls in Gegenwart eines Verdünnungsmittels umsetzt,
und gegebenenfalls die nach den Verfahren (a), (b), (c), (d) oder (e) erhaltenen Verbindungen der Formel (I) nach üblichen Methoden in Salze überführt.

4. Herbizide Mittel, **gekennzeichnet durch** einen Gehalt an mindestens einer Verbindung der Formel (I) oder einem ihrer Salze gemäß Anspruch 1.

5. Verwendung von Verbindungen der allgemeinen Formel (I) oder deren Salzen gemäß Anspruch 1 zur Bekämpfung von unerwünschtem Pflanzenwachstum.

6. Verfahren zur Bekämpfung von Unkräutern, **dadurch gekennzeichnet, dass** man Verbindungen der allgemeinen Formel (I) oder deren Salze gemäß Anspruch 1 auf die Unkräuter oder ihren Lebensraum einwirken lässt.

7. Verfahren zur Herstellung von herbiziden Mitteln, **dadurch gekennzeichnet, dass** man Verbindungen der allgemeinen Formel (I) oder deren Salze gemäß Anspruch 1 mit Streckmitteln und/oder oberflächenaktiven Mitteln vermischt.

8. Sulfonamide der allgemeinen Formel (II), 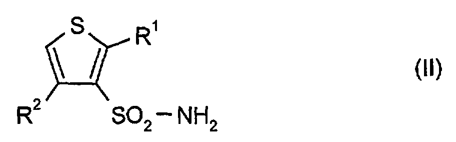 in welcher
R¹ und R² die in Anspruch 1 angegebenen Bedeutungen haben.

9. Sulfonyliso(thio)cyanate der allgemeinen Formel (IV), 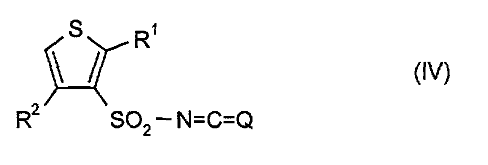 in welcher
Q, R¹ und R² die in Anspruch 1 angegebenen Bedeutungen haben.

10. Sulfonsäurechloride der allgemeinen Formel (VI), 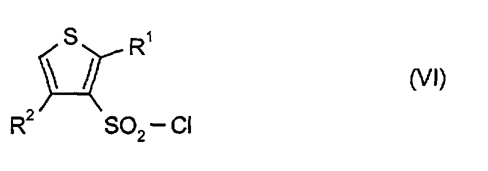 in welcher
R¹ und R² die in Anspruch 1 angegebenen Bedeutungen haben.

## Claims

1. Thienylsulphonylamino(thio)carbonyl compounds of the general formula (I) 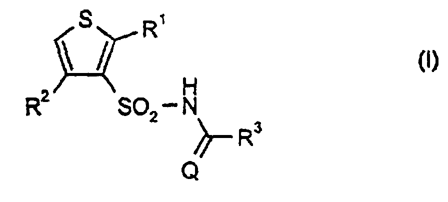 in which
Q represents oxygen or sulphur,
R¹ represents cyano, halogen or in each case optionally cyano-, halogen- or C₁-C₄-alkoxy-substituted alkyl, alkenyl, alkinyl, alkoxy, alkenyloxy or alkinyloxy having in each case up to 6 carbon atoms,
R² represents cyano, halogen or in each case optionally cyano-, halogen- or C₁-C₄-alkoxy-substituted alkyl, alkenyl, alkinyl, alkoxy, alkenyloxy or alkinyloxy having in each case up to 6 carbon atoms, and
R³ represents in each case optionally substituted heterocyclyl of the formulae below 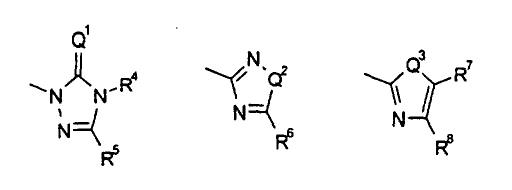
in which
Q¹, Q² and Q³ each represent oxygen or sulphur and
R⁴ represents hydrogen, hydroxyl, amino, cyano, represents C₂-C₁₀-alkylideneamino, represents optionally fluorine-, chlorine-, bromine-, cyano-, C₁-C₄-alkoxy-, C₁-C₄-alkyl-carbonyl- or C₁-C₄-alkoxy-carbonyl-substituted C₁-C₆-alkyl, represents in each case optionally fluorine-, chlorine- and/or bromine-substituted C₂-C₆-alkenyl or C₂-C₆-alkinyl, represents in each case optionally fluorine-, chlorine-, bromine-, cyano-, C₁-C₄-alkoxy- or C₁-C₄-alkoxy-carbonyl-substituted C₁-C₆-alkoxy, C₁-C₆-alkylamino or C₁-C₆-alkyl-carbonylamino, represents C₃-C₆-alkenyloxy, represents di-(C₁-C₄-alkyl)-amino, represents in each case optionally fluorine-, chlorine-, bromine-, cyano- and/or C₁-C₄-alkyl-substituted C₃-C₆-cycloalkyl, C₃-C₆-cycloalkylamino or C₃-C₆-cycloalkyl-C₁-C₄-alkyl, or represents in each case optionally fluorine-, chlorine-, bromine-, cyano-, nitro-, C₁-C₄-alkyl-, trifluoromethyl and/or C₁-C₄-alkoxy-substituted phenyl or phenyl-C₁-C₄-alkyl,
R⁵ represents hydrogen, hydroxyl, mercapto, amino, cyano, fluorine, chlorine, bromine, iodine, represents optionally fluorine-, chlorine-, bromine-, cyano-, C₁-C₄-alkoxy-, C₁-C₄-alkyl-carbonyl- or C₁-C₄-alkoxy-carbonyl-substituted C₁-C₆-alkyl, represents in each case optionally fluorine-, chlorine- and/or bromine-substituted C₂-C₆-alkenyl or C₂-C₆-alkinyl, represents in each case optionally fluorine-, chlorine-, cyano-, C₁-C₄-alkoxy- or C₁-C₄-alkoxycarbonyl-substituted C₁-C₆-alkoxy, C₁-C₆-alkylthio, C₁-C₆-alkylamino or C₁-C₆-alkyl-carbonylamino, represents C₃-C₆-alkenyloxy, C₃-C₆-alkinyloxy, C₃-C₆-alkenylthio, C₃-C₆-alkinylthio, C₃-C₆-alkenylamino or C₃-C₆-alkinylamino, represents di-(C₁-C₄-alkyl)-amino, represents in each case optionally methyl- and/or ethyl-substituted aziridino, pyrrolidino, piperidino or morpholino, represents in each case optionally fluorine-, chlorine-, bromine-, cyano- and/or C₁-C₄-alkyl-substituted C₃-C₆-cycloalkyl, C₅-C₆-cycloalkenyl, C₃-C₆-cycloalkyloxy, C₃-C₆-cycloalkylthio, C₃-C₆-cycloalkylamino, C₃-C₆-cycloalkyl-C₁-C₄-alkyl, C₃-C₆-cycloalkyl-C₁-C₄-alkoxy, C₃-C₆-cycloalkyl-C₁-C₄-alkylthio or C₃-C₆-cycloalkyl-C₁-C₄-alkylamino, or represents in each case optionally fluorine-, chlorine-, bromine-, cyano-, nitro-, C₁-C₄-alkyl-, trifluoromethyl-, C₁-C₄-alkoxy- and/or C₁-C₄-alkoxycarbonyl-substituted phenyl, phenyl-C₁-C₄-alkyl, phenoxy, phenyl-C₁-C₄-alkoxy, phenylthio, phenyl-C₁-C₄-alkylthio, phenylamino or phenyl-C₁-C₄-alkylamino, or
R⁴ and R⁵ together represent optionally branched alkanediyl having 3 to 11 carbon atoms, furthermore
R⁶, R⁷ and R⁸ are identical or different and each represent hydrogen, cyano, fluorine, chlorine, bromine, or represent in each case optionally fluorine-, chlorine-, bromine- or C₁-C₄-alkoxy-substituted alkyl, alkenyl, alkinyl, alkoxy, alkenyloxy, alkinyloxy, alkylthio, alkenylthio, alkinylthio, alkylsulphinyl or alkylsulphonyl having in each case up to 6 carbon atoms or represent optionally cyano-, fluorine-, chlorine-, bromine or C₁-C₄-alkyl-substituted cycloalkyl having 3 to 6 carbon atoms,
and salts of compounds of the formula (I).

2. Compounds of the formula (I) according to Claim 1, **characterized in that**
Q represents oxygen or sulphur,
R¹ represents cyano, halogen, represents optionally cyano-, halogen- or C₁-C₄-alkoxy-substituted C₁-C₄-alkyl, represents in each case optionally cyano- or halogen-substituted C₂-C₄-alkenyl or C₂-C₄-alkinyl, represents optionally cyano-, halogen- or C₁-C₄-alkoxy-substituted C₁-C₄-alkoxy, or represents in each case optionally cyano- or halogen-substituted C₂-C₄-alkenyloxy or C₂-C₄-alkinyloxy,
R² represents cyano, halogen, represents optionally cyano-, halogen- or C₁-C₄-alkoxy-substituted C₁-C₄-alkyl, represents in each case optionally cyano- or halogen-substituted C₂-C₄-alkenyl or C₂-C₄-alkinyl, represents optionally cyano-, halogen- or C₁-C₄-alkoxy-substituted C₁-C₄-alkoxy, or represents in each case optionally cyano- or halogen-substituted C₂-C₄-alkenyloxy or C₂-C₄-alkinyloxy, and
R³ represents in each case optionally substituted heterocyclyl of the formulae below 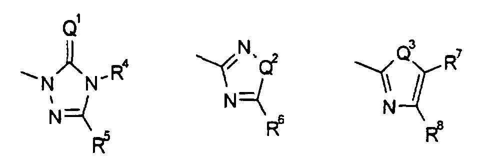
in which
Q¹, Q² and Q³ each represent oxygen or sulphur and
R⁴ represents hydrogen, hydroxyl, amino, cyano, represents C₂-C₁₀-alkylideneamino, represents optionally fluorine-, chlorine-, bromine-, cyano-, C₁-C₄-alkoxy-, C₁-C₄-alkyl-carbonyl- or C₁-C₄-alkoxy-carbonyl-substituted C₁-C₆-alkyl, represents in each case optionally fluorine-, chlorine- and/or bromine-substituted C₂-C₆-alkenyl or C₂-C₆-alkinyl, represents in each case optionally fluorine-, chlorine-, bromine-, cyano-, C₁-C₄-alkoxy- or C₁-C₄-alkoxy-carbonyl-substituted C₁-C₆-alkoxy, C₁-C₆-alkylamino or C₁-C₆-alkyl-carbonylamino, represents C₃-C₆-alkenyloxy, represents di-(C₁-C₄-alkyl)-amino, represents in each case optionally fluorine-, chlorine-, bromine-, cyano- and/or C₁-C₄-alkyl-substituted C₃-C₆-cycloalkyl, C₃-C₆-cycloalkylamino or C₃-C₆-cycloalkyl-C₁-C₄-alkyl, or represents in each case optionally fluorine-, chlorine-, bromine-, cyano-, nitro-, C₁-C₄-alkyl-, trifluoromethyl and/or C₁-C₄-alkoxy-substituted phenyl or phenyl-C₁-C₄-alkyl,
R⁵ represents hydrogen, hydroxyl, mercapto, amino, cyano, fluorine, chlorine, bromine, iodine, represents optionally fluorine-, chlorine-, bromine-, cyano-, C₁-C₄-alkoxy-, C₁-C₄-alkyl-carbonyl- or C₁-C₄-alkoxy-carbonyl-substituted C₁-C₆-alkyl, represents in each case optionally fluorine-, chlorine- and/or bromine-substituted C₂-C₆-alkenyl or C₂-C₆-alkinyl, represents in each case optionally fluorine-, chlorine-, cyano-, C₁-C₄-alkoxy- or C₁-C₄-alkoxycarbonyl-substituted C₁-C₆-alkoxy, C₁-C₆-alkylthio, C₁-C₆-alkylamino or C₁-C₆-alkyl-carbonylamino, represents C₃-C₆-alkenyloxy, C₃-C₆-alkinyloxy, C₃-C₆-alkenylthio, C₃-C₆-alkinylthio, C₃-C₆-alkenylamino or C₃-C₆-alkinylamino, represents di-(C₁-C₄-alkyl)-amino, represents in each case optionally methyl- and/or ethyl-substituted aziridino, pyrrolidino, piperidino or morpholino, represents in each case optionally fluorine-, chlorine-, bromine-, cyano- and/or C₁-C₄-alkyl-substituted C₃-C₆-cycloalkyl, C₅-C₆-cycloalkenyl, C₃-C₆-cycloalkyloxy, C₃-C₆-cycloalkylthio, C₃-C₆-cycloalkylamino, C₃-C₆-cycloalkyl-C₁-C₄-alkyl, C₃-C₆-cycloalkyl-C₁-C₄-alkoxy, C₃-C₆-cycloalkyl-C₁-C₄-alkylthio or C₃-C₆-cycloalkyl-C₁-C₄-alkylamino, or represents in each case optionally fluorine-, chlorine-, bromine-, cyano-, nitro-, C₁-C₄-alkyl-, trifluoromethyl-, C₁-C₄-alkoxy- and/or C₁-C₄-alkoxycarbonyl-substituted phenyl, phenyl-C₁-C₄-alkyl, phenoxy, phenyl-C₁-C₄-alkoxy, phenylthio, phenyl-C₁-C₄-alkylthio, phenylamino or phenyl-C₁-C₄-alkylamino, or
R⁴ and R⁵ together represent optionally branched alkanediyl having 3 to 11 carbon atoms, furthermore
R⁶, R⁷ and R⁸ are identical or different and each represent hydrogen, cyano, fluorine, chlorine, bromine, or represent in each case optionally fluorine-, chlorine-, bromine- or C₁-C₄-alkoxy-substituted alkyl, alkenyl, alkinyl, alkoxy, alkenyloxy, alkinyloxy, alkylthio, alkenylthio, alkinylthio, alkylsulphinyl or alkylsulphonyl having in each case up to 6 carbon atoms or represent optionally cyano-, fluorine-, chlorine-, bromine or C₁-C₄-alkyl-substituted cycloalkyl having 3 to 6 carbon atoms,
and the sodium, potassium, magnesium, calcium, ammonium, C₁-C₄-alkylammonium, di-(C₁-C₄-alkyl)-ammonium, tri-(C₁-C₄-alkyl)-ammonium, tetra-(C₁-C₄-alkyl)-ammonium, tri-(C₁-C₄-alkyl)-sulphonium, C₅- or C₆-cycloalkyl-ammonium and di-(C₁-C₂-alkyl)-benzyl-ammonium salts of compounds of the formula (I).

3. Process for preparing thienylsulphonylamino(thio)carbonyl compounds of the general formula (I) according to Claim 1, **characterized in that**
(a) sulphonamides of the general formula (II), 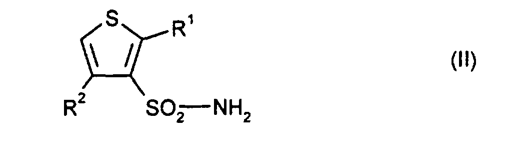 in which
R¹ and R² are each as defined in Claim 1,
are reacted with (thio)carboxylic acid derivatives of the general formula (III), 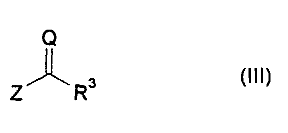 in which
Q and R³ are each as defined in Claim 1 and
Z represents halogen, alkoxy, aryloxy or arylalkoxy,
if appropriate in the presence of an acid acceptor and if appropriate in the presence of a diluent,
or **in that**
(b) sulphonyl iso(thio)cyanates of the general formula (IV) 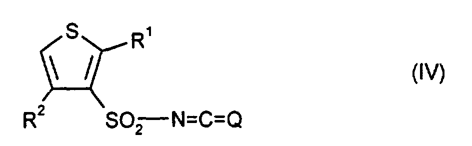 in which
Q, R¹ and R² are each as defined above,
are reacted with heterocycles of the general formula (V)
H-R³⁻¹ (V)
in which
R³⁻¹ represents optionally substituted triazolinyl of the formula below 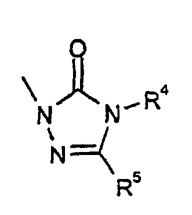
in which Q represents oxygen or sulfphur and the radicals R⁴ and R⁵ are each as defined above,
if appropriate in the presence of a reaction auxiliary and if appropriate in the presence of a diluent,
or **in that**
(c) sulphonyl chlorides of the general formula (VI) 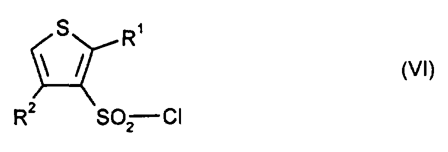 in which
R¹ and R² are each as defined above,
are reacted with heterocycles of the general formula (V)
H-R³⁻¹ (V)
in which
R³⁻¹ is as defined above under (b),
and metal (thio)cyanates of the general formula (VII)
MQCN (VII)
in which
Q is as defined above and
M represents a metal equivalent,
if appropriate in the presence of a reaction auxiliary and if appropriate in the presence of a diluent,
or **in that**
(d) sulphonyl chlorides of the general formula (VI) 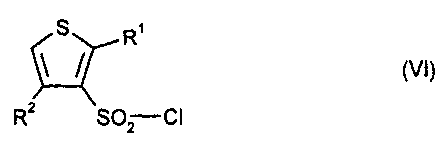 in which
R¹ and R² are each as defined above,
are reacted with (thio)carboxamides of the general formula (VIII) 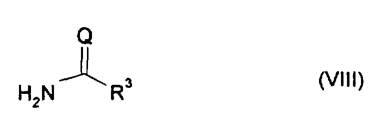 in which
Q and R³ are each as defined above under formula (I) in Claim 1, if appropriate in the presence of an acid acceptor and if appropriate in the presence of a diluent,
or **in that**
(e) sulphonylamino(thio)carbonyl compounds of the general formula (IX) 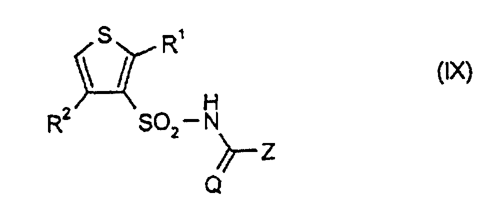 in which
Q, R¹ and R² are each as defined above and
Z represents halogen, alkoxy, aryloxy or arylalkoxy,
are reacted with heterocycles of the general formula (V)
H-R³⁻¹ (V)
in which
R³⁻¹ is as defined above under (b),
if appropriate in the presence of an acid acceptor and if appropriate in the presence of a diluent,
and the compounds of the formula (I) obtained by the processes (a), (b), (c), (d) or (e) are, if appropriate, converted into salts by customary methods.

4. Herbicidal compositions, **characterized in that** they contain at least one compound of the formula (I) or a salt thereof according to Claim 1.

5. The use of compounds of the general formula (I) or salts thereof according to Claim 1 for controlling undesirable vegetation.

6. Method for controlling weeds, **characterized in that** compounds of the general formula (I) or salts thereof according to Claim 1 are allowed to act on the weeds or their habitat.

7. Process for preparing herbicidal compositions, **characterized in that** compounds of the general formula (I) or salts thereof according to Claim 1 are mixed with extenders and/or surfactants.

8. Sulphonamides of the general formula (II), 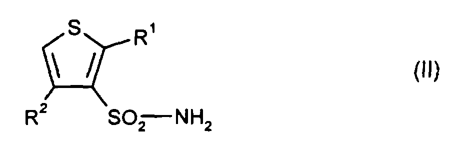 in which
R¹ and R² are each as defined in Claim 1.

9. Sulphonyl iso(thio)cyanates of the general formula (IV), 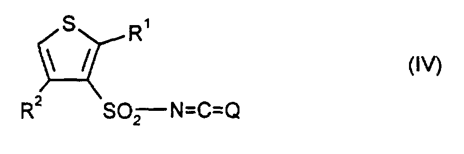 in which
Q, R¹ and R² are each as defined in Claim 1.

10. Sulphonyl chlorides of the general formula (VI), 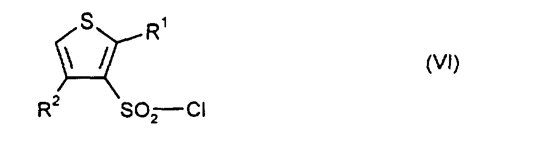 in which
R¹ and R² are each as defined in Claim 1.

## Revendications

1. Composés thiénylsulfonylamino (thio)-carbonylés de formule générale (I) : 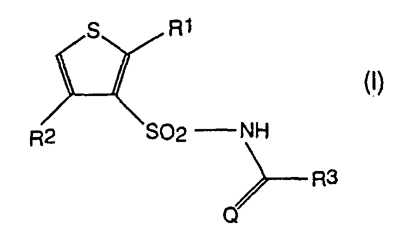 dans laquelle :
Q représente l'atome d'oxygène ou de soufre ;
R¹ représente le radical cyano, un atome d'halogène ou les radicaux alcoyle, alcényle, alcynyle, alcoxy, alcényloxy ou alcynyloxy, ayant chacun jusqu'à 6 atomes de carbone, chaque fois éventuellement substitués par le radical cyano, un atome d'halogène ou le radical alcoxy en C₁-C₄ ;
R² représente le radical cyano, un atome d'halogène ou les radicaux alcoyle, alcényle, alcynyle, alcoxy, alcényloxy ou alcynyloxy, ayant chacun jusqu'à 6 atomes de carbone, chaque fois éventuellement substitués par le radical cyano, un atome d'halogène ou le radical alcoxy en C₁-C₄, et
R³ représente un radical hétérocyclyle, chaque fois éventuellement substitué, des formules suivantes : 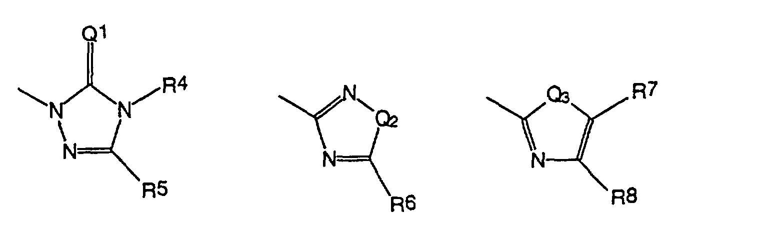
où :
Q¹, Q² et Q³ représentent chaque fois, l'atome d'oxygène ou de soufre, ainsi que
R⁴ représente l'atome d'hydrogène, les radicaux hydroxy, amino, cyano, le radical (alcoylidène en C₂-C₁₀)amino, le radical alcoyle en C₁-C₆ éventuellement substitué par les atomes de fluor, de chlore, de brome, les radicaux cyano, alcoxy en C₁-C₄, (alcoyl en C₁-C₄)carbonyle ou (alcoxy en C₁-C₄)carbonyle, les radicaux alcényle en C₂-C₆ ou alcynyle en C₂-C₆, chaque fois éventuellement substitués par les atomes de fluor, de chlore et/ou de brome, les radicaux alcoxy en C₁-C₆, alcoylamino en C₁-C₆ ou (alcoyl en C₁-C₆)carbonylamino, chaque fois éventuellement substitués par les atomes de fluor, de chlore, de brome, les radicaux cyano, alcoxy en C₁-C₄ ou (alcoxy en C₁-C₄)carbonyle, le radical alcényloxy en C₃-C₆, le radical di (alcoyl en C₁-C₄) amino, les radicaux cycloalcoyle en C₃-C₆, cycloalcoylamino en C₃-C₆ ou (cycloalcoyl en C₃-C₆)alcoyle en C₁-C₄, chaque fois éventuellement substitués par les atomes de fluor, de chlore, de brome, les radicaux cyano et/ou alcoyle en C₁-C₄, ou les radicaux phényle ou phényl(alcoyle en C₁-C₄), chaque fois éventuellement substitués par les atomes de fluor, de chlore, de brome, les radicaux cyano, nitro, alcoyle en C₁-C₄, trifluorométhyle et/ou alcoxy en C₁-C₄ ;
R⁵ représente l'atome d'hydrogène, les radicaux hydroxy, mercapto, amino, cyano, les atomes de fluor, de chlore, de brome, d'iode, le radical alcoyle en C₁-C6 éventuellement substitué par les atomes de fluor, de chlore, de brome, les radicaux cyano, alcoxy en C₁-C₄, (alcoyl en C₁-C₄) carbonyle ou (alcoxy en C₁-C₄)carbonyle, les radicaux alcényle en C₂-C₆ ou alcynyle en C₂-C₆, chaque fois éventuellement substitués par les atomes de fluor, de chlore et/ou de brome, les radicaux alcoxy en C₁-C₆, alcoylthio en C₁-C₆, alcoylamino en C₁-C₆ ou (alcoyl en C₁-C₆)carbonylamino, chaque fois éventuellement substitués par les atomes de fluor, de chlore, les radicaux cyano, alcoxy en C₁-C₄ ou (alcoxy en C₁-C₄)carbonyle, les radicaux alcényloxy en C₃-C₆, alcynyloxy en C₃-C₆, alcénylthio en C₃-C₆, alcynylthio en C₃-C₆, alcénylamino en C₃-C₆ ou alcynylamino en C₃-C₆, le radical di(alcoyl en C₁-C₄)amino, les radicaux aziridino, pyrrolidino, pipéridino ou morpholino, chaque fois éventuellement substitués par les radicaux méthyle et/ou éthyle, les radicaux cycloalcoyle en C₃-C₆, cycloalcényle en C₅-C₆, cycloalcoyloxy en C₃-C₆, cycloalcoylthio en C₃-C₆, cycloalcoylamino en C₃-C₆, (cycloalcoyl en C₃-C₆)alcoyle en C₁-C₄, (cycloalcoyl en C₃-C₆)alcoxy en C₁-C₄, (cycloalcoyl en C₃-C₆)alcoylthio en C₁-C₄ ou (cycloalcoyl en C₃-C₆)alcoylamino en C₁-C₄, chaque fois éventuellement substitués par les atomes de fluor, de chlore, de brome, les radicaux cyano et/ou alcoyle en C₁-C₄, ou les radicaux phényle, phényl(alcoyle en C₁-C₄), phénoxy, phényl(alcoxy en C₁-C₄), phénylthio, phényl (alcoylthio en C₁-C₄), phénylamino, phényl(alcoylamino en C₁-C₄), chaque fois éventuellement substitués par les atomes de fluor, de chlore, de brome, les radicaux cyano, nitro, alcoyle en C₁-C₄, trifluorométhyle, alcoxy en C₁-C₄ et/ou (alcoxy en C₁-C₄) carbonyle, ou
R⁴ et R⁵ représentent ensemble, un radical alcandiyle éventuellement ramifié, ayant 3 à 11 atomes de carbone, en outre
R⁶, R⁷ et R⁸ sont identiques ou différents et représentent l'atome d'hydrogène, le radical cyano, les atomes de fluor, de chlore, de brome, ou les radicaux alcoyle, alcényle, alcynyle, alcoxy, alcényloxy, alcynyloxy, alcoylthio, alcénylthio, alcynylthio, alcoylsulfinyle ou alcoylsulfonyle, chaque fois éventuellement substitués par les atomes de fluor, de chlore, de brome ou le radical alcoxy en C₁-C₄, ayant chaque fois jusqu'à 6 atomes de carbone, ou le radical cycloalcoyle éventuellement substitué par le radical cyano, les atomes de fluor, de chlore, de brome ou un radical alcoyle en C₁-C₄, ayant 3 à 6 atomes de carbone,
ainsi que les sels des composés de formule (I).

2. Composés de formule (I) suivant la revendication 1, **caractérisés en ce que** :
Q représente l'atome d'oxygène ou de soufré ;
R¹ représente le radical cyano, un atome d'halogène ou le radical alcoyle en C₁-C₄, éventuellement substitué par le radical cyano, un atome d'halogène ou le radical alcoxy en C₁-C₄, les radicaux alcényle en C₂-C₄ ou alcynyle en C₂-C₄, chaque fois éventuellement substitués par le radical cyano ou un atome d'halogène, len radical alcoxy en C₁-C₄, éventuellement substitué par le radical cyano, un atome d'halogène ou le radical alcoxy en C₁-C₄ ou les radicaux alcényloxy en C₂-C₄ ou alcynyloxy en C₂-C₄, chaque fois éventuellement substitués par le radical cyano ou un atome d'halogène ;
R² représente le radical cyano, un atome d'halogène ou le radical alcoyle en C₁-C₄, éventuellement substitué par le radical cyano, un atome d'halogène ou le radical alcoxy en C₁-C₄, les radicaux alcényle en C₂-C₄ ou alcynyle en C₂-C₄, chaque fois éventuellement substitués par le radical cyano ou un atome d'halogène, le radical alcoxy en C₁-C₄, éventuellement substitué par le radical cyano, un atome d'halogène ou le radical alcoxy en C₁-C₄ ou les radicaux alcényloxy en C₂-C₄ ou alcynyloxy en C₂-C₄, chaque fois éventuellement substitués par le radical cyano ou un atome d'halogène ; et
R³ représente un radical hétérocyclyle, chaque fois éventuellement substitué, des formules suivantes : 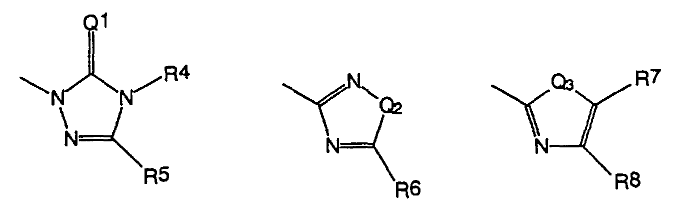
où :
Q¹, Q² et Q³ représentent chaque fois, l'atome d'oxygène ou de soufre, ainsi que
R⁴ représente l'atome d'hydrogène, les radicaux hydroxy, amino, cyano, le radical (alcoylidène en C₂-C₁₀)amino, le radical alcoyle en C₁-C₆ éventuellement substitué par les atomes de fluor, de chlore, de brome,. les radicaux cyano, alcoxy en C₁-C₄, (alcoylen C₁-C₄) carbonyle ou (alcoxy en C₁-C₄) carbonyle, les radicaux alcényle en C₂-C₆ ou alcynyle en C₂-C₆, chaque fois éventuellement substitués par les atomes de fluor, de chlore et/ou de brome, les radicaux alcoxy en C₁-C₆, alcoylamino en C₁-C₆ ou (alcoyl en C₁-C₆)carbonylamino, chaque fois éventuellement substitués par les atomes de fluor, de chlore, de brome, les radicaux cyano, alcoxy en C₁-C₄ ou (alcoxy en C₁-C₄)carbonyle, le radical alcényloxy en C₃-C₆, le radical di (alcoyl en C₁-C₄) amino, les radicaux cycloalcoyle en C₃-C₆, cycloalcoylamino en C₃-C₆ ou (cycloalcoyl en C₃-C₆)alcoyle en C₁-C₄, chaque fois éventuellement substitués par les atomes de fluor, de chlore, de brome, les radicaux cyano et/ou alcoyle en C₁-C₄, ou les radicaux phényle ou phényl(alcoyle en C₁-C₄), chaque fois éventuellement substitués par les atomes de fluor, de chlore, de brome, les radicaux cyano, nitro, alcoyle en C₁-C₄, trifluorométhyle et/ou alcoxy en C₁-C₄ ;
R⁵ représente l'atome d'hydrogène, les radicaux hydroxy, mercapto, amino, cyano, les atomes de fluor, de chlore, de brome, d'iode, le radical alcoyle en C₁-C₆ éventuellement substitué par les atomes de fluor, de chlore, de brome, les radicaux cyano, alcoxy en C₁-C₄, (alcoyl en C₁-C₄) carbonyle ou (alcoxy en C₁C₄)carbonyle, les radicaux alcényle en C₂-C₆ ou alcynyle en C₂-C₆, chaque fois éventuellement substitués par les atomes de fluor, de chlore et/ou de brome, les radicaux alcoxy en C₁-C₆, alcoylthio en C₁-C₆, alcoylamino en C₁-C₆ ou (alcoyl en C₁-C₆) carbonylamino, chaque fois éventuellement substitués par les atomes de fluor, de chlore, les radicaux cyano, alcoxy en C₁-C₄ ou (alcoxy en C₁-C₄)carbonyle, les radicaux alcényloxy en C₃-C₆, alcynyloxy en C₃-C₆, alcénylthio en C₃-C₆, alcynylthio en C₃-C₆, alcénylamino en C₃-C₆ ou alcynylamino en C₃-C₆, le radical di(alcoyl en C₁-C₄)amino, les radicaux aziridino, pyrrolidino, pipéridino ou morpholino, chaque fois éventuellement substitués par les radicaux méthyle et/ou éthyle, les radicaux cycloalcoyle en C₃-C₆, cycloalcényle en C₅-C₆, cycloalcoyloxy en C₃-C₆, cycloalcoylthio en C₃-C₆, cycloalcoylamino en C₃-C₆, (cycloalcoyl en C₃-C₆)alcoyle en C₁-C₄, (cycloalcoyl en C₃-C₆)alcoxy en C₁-C₄, (cycloalcoyl en C₃-C₆)alcoylthio en C₁-C₄ ou (cycloalcoyl en C₃-C₆)alcoylamino en C₁-C₄, chaque fois éventuellement substitués par les atomes de fluor, de chlore, de brome, les radicaux cyano et/ou alcoyle en C₁-C₄, ou les radicaux phényle, phényl(alcoyle en C₁-C₄), phénoxy, phényl(alcoxy en C₁C₄), phénylthio, phényl(alcoylthio en C₁-C₄), phénylamino, phényl(alcoylamino en C₁-C₄), chaque fois éventuellement substitués par les atomes de fluor, de chlore, de brome, les radicaux cyano, nitro, alcoyle en C₁-C₄, trifluorométhyle, alcoxy en C₁-C₄ et/ou (alcoxy en C₁-C₄) carbonyle, ou
R⁴ et R⁵ représentent ensemble, un radical alcandiyle éventuellement ramifié, ayant 3 à 11 atomes de carbone, en outre
R⁶, R⁷ et R⁸ sont identiques ou différents et représentent l'atome d'hydrogène, le radical cyano, les atomes de fluor, de chlore, de brome, ou les radicaux alcoyle, alcényle, alcynyle, alcoxy, alcényloxy, alcynyloxy, alcoylthio, alcénylthio, alcynylthio, alcoylsulfinyle ou alcoylsulfonyle, chaque fois éventuellement substitués par les atomes de fluor, de chlore, de brome ou le radical alcoxy en C₁-C₄, ayant chaque fois jusqu'à 6 atomes de carbone, ou le radical cycloalcoyle éventuellement substitué par le radical cyano, les atomes de fluor, de chlore, de brome ou le radical alcoyle en C₁-C₄, ayant 3 à 6 atomes de carbone,
ainsi que les sels de sodium, de potassium, de magnésium, de calcium, d'(alcoyl en C₁-C₄)ammonium, de di (alcoyl en C₁-C₄)ammonium, de tri(alcoyl en C₁C₄) ammonium, de tétra(alcoyl en C₁-C₄)ammonium, de tri(alcoyl en C₁-C₄)sulfonium, de (cycloalcoyl en C₅ ou C₆)ammonium et de di(alcoyl en C₁-C₂)benzylammonium des composés de formule (I).

3. Procédé de préparation de composés thiénylsulfonylamino(thio)carbonylés de formule générale (I) suivant la revendication 1, **caractérisé en ce que** l'on :
(a) fait réagir des sulfonamides de formule générale (II) : 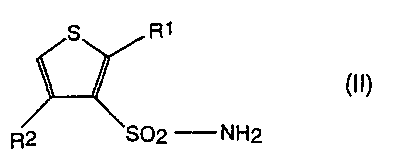 dans laquelle :
R¹ et R² ont la signification donnée à la revendication 1,
avec des dérivés acide (thio)carboxyliques de formule générale (III) : 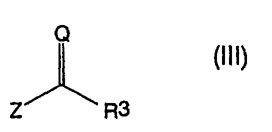 dans laquelle :
Q et R³ ont la signification donnée à la revendication 1, et
Z représente un atome d'halogène, les radicaux alcoxy, aryloxy ou arylalcoxy,
le cas échéant en présence d'un accepteur d'acide et le cas échéant en présence d'un agent de dilution,
ou lorsque l'on
(b) fait réagir des sulfonyliso(thio)cyanates de formule générale (IV) : 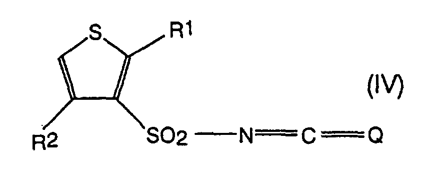 dans laquelle :
Q, R¹ et R² ont la signification donnée ci-dessus,
avec des hétérocycles de formule générale (V) :
H - R³⁻¹ (V)
dans laquelle :
R³⁻¹ représente le radical triazolinyle éventuellement substitué de la formule suivante : 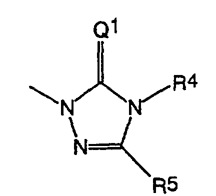
où Q¹ représente l'atome d'oxygène ou de soufre et les restes R⁴ et R⁵ ont la signification donnée ci-dessus,
le cas échéant en présence d'un auxiliaire de réaction et le cas échéant en présence d'un agent de dilution,
ou lorsque l'on
(c) fait réagir des chlorures d'acide sulfonique de formule générale (VI) : 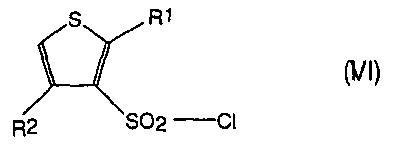 dans laquelle :
R¹ et R² ont la signification donnée ci-dessus, avec des hétérocycles de formule générale (V) :
H - R³⁻¹ (V)
dans laquelle :
R³⁻¹ a la signification donnée ci-dessus sous (b),
avec des (thio)cyanates métalliques de formule générale (VII) :
MQCN (VII)
dans laquelle :
Q a la signification donnée ci-dessus,
M représente un équivalent métallique,
le cas échéant en présence d'un auxiliaire de réaction et le cas échéant en présence d'un agent de dilution,
ou lorsque l'on
(d) fait réagir des chlorures d'acide sulfonique de formule générale (VI) : 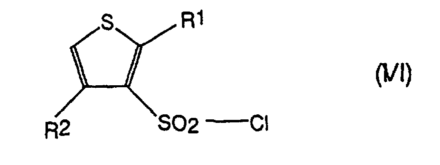 dans laquelle :
R¹ et R² ont la signification donnée ci-dessus,
avec des amides d'acide (thio)carboxylique de formule générale (VIII) : 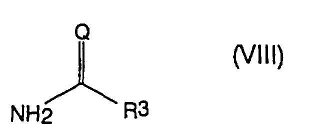
dans laquelle :
Q et R³ ont la signification donnée à la revendication 1 pour la formule (I),
le cas échéant en présence d'un accepteur d'acide et le cas échéant en présence d'un agent de dilution,
ou lorsque l'on
(e) fait réagir des composés sulfonylamino-(thio)carbonylés de formule générale (IX) : 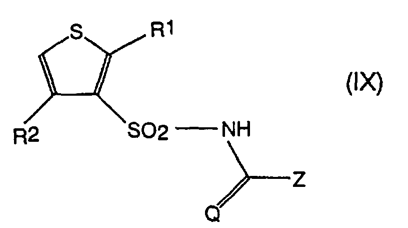
dans laquelle :
Q, R¹ et R² ont la signification donnée ci-dessus, et
Z représente un atome d'halogène, les radicaux alcoxy, aryloxy ou arylalcoxy,
avec des hétérocycles de formule générale (V) :
H - R³⁻¹ (V)
dans laquelle :
R³⁻¹ a la signification donnée ci-dessus sous (b),
le cas échéant en présence d'un accepteur d'acide et le cas échéant en présence d'un agent de dilution,
et le cas échéant, les composés de formule (I), obtenus suivant les procédés (a), (b), (c), (d) ou (e), sont convertis en sels par des procédés usuels.

4. Agent herbicide, **caractérisé par** une teneur en au moins un composé de formule (I) ou un de ses sels, suivant la revendication 1.

5. Utilisation des composés de formule générale (I) ou de leurs sels, suivant la revendication 1, pour lutter contre la croissance non souhaitée de plantes.

6. Procédé pour lutter contre les mauvaises herbes, **caractérisé en ce que** l'on fait agir des composés de formule générale (I) ou leurs sels, suivant la revendication 1, sur les mauvaises herbes ou leur site de développement.

7. Procédé de préparation d'agents herbicides, **caractérisé en ce que** l'on mélange des composés de formule générale (I) ou leurs sels, suivant la revendication 1, avec un agent de dilution et/ou un agent tensioactif.

8. Sulfonamide de formule générale (II) : 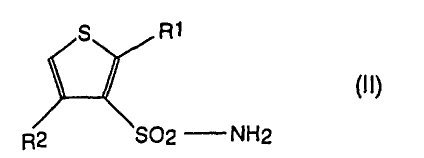 dans laquelle :
R¹ et R² ont la signification donnée à la revendication 1.

9. Sulfonyliso(thio)cyanate de formule générale (IV) : 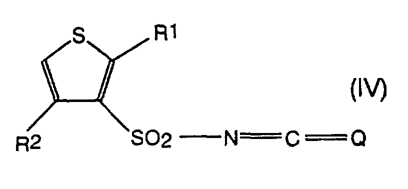 dans laquelle :
Q, R¹ et R² ont la signification donnée à la revendication 1.

10. Chlorure d'acide sulfonique de formule générale (VI) : 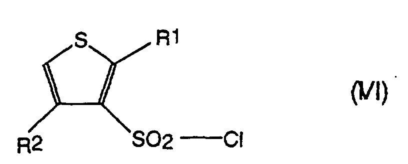 dans laquelle :
R¹ et R² ont la signification donnée à la revendication 1.
